# FASCICULE DE BREVET EUROPEEN

(11) **EP 3 191 221 B1**
(45) Date de publication et mention de la délivrance du brevet: **01.01.2020**
(21) Numéro de dépôt: 15759689.1
(22) Date de dépôt: 21.08.2015
(51) Int. Cl.: B01J 21/04, B01J 23/755, B01J 23/78, B01J 23/835, B01J 23/89, B01J 27/185, B01J 33/00, B01J 37/03, B01J 37/08, B01J 37/16, B01J 37/20, B01J 35/10, B01J 37/02, C07C 5/03, C07C 5/10, C10G 45/36, C10G 45/48, C01F 7/02, C01F 7/34

(54) **CATALYSEUR MESOPOREUX A BASE DE NICKEL ET SON UTILISATION EN HYDROGENATION D'HYDROCARBURES**
MESOPORÖSER NICKEL-HALTIGER KATALYSATOR UND DESSEN VERWENDUNG ZUR HYDRIERUNG VON KOHLENWASSERSTOFFEN
MESOPOROUS NICKEL-BASED CATALYST AND USE THEREOF FOR HYDROCARBON HYDROGENATION

(30) Priorité: 11.09.2014 FR 1458543
(43) Date de publication de la demande: 19.07.2017
(73) Titulaire: IFP Energies nouvelles, 92500 Rueil-Malmaison (FR)
(72) Inventeur: BOUALLEG, Malika, F-69100 Villeurbanne (FR); DUBREUIL, Anne-Claire, F-69003 Lyon (FR); MAILLE, Emily, F-69004 Lyon (FR); THOMAZEAU, Cecile, F-69008 Lyon (FR)
(74) Mandataire: IFP Energies nouvelles
(86) Numéro de dépôt international: PCT/EP2015/069223
(87) Numéro de publication internationale: WO 2016/037830

(56) Documents cités:
- EP-A1- 0 168 091
- EP-A1- 0 885 844
- WO-A1-2005/028106
- US-A- 5 478 791
- US-B1- 6 589 908

## Description

### Domaine de l'invention

L'invention a pour objet un catalyseur supporté sur un support oxyde majoritairement aluminique calciné à phase active de nickel présentant une texture et une formulation favorables aux réactions d'hydrogénation, notamment aux réactions d'hydrogénation sélective de composés polyinsaturés ou d'hydrogénation d'aromatiques. L'invention concerne également le procédé de préparation dudit catalyseur ainsi que son utilisation dans des réactions d'hydrogénation.

Les catalyseurs d'hydrogénation sélective ou d'hydrogénation d'aromatiques sont généralement à base de métaux du groupe VIII de la classification périodique des éléments tel que le nickel. Le métal se présente sous la forme de particules métalliques nanométriques déposées sur un support qui peut être un oxyde réfractaire. La teneur en métal du groupe VIII, la présence éventuelle d'un deuxième élément métallique, la taille des particules de métal et la répartition de la phase active dans le support ainsi que la nature et distribution poreuse du support sont des paramètres qui ont une importance sur les performances des catalyseurs.

La vitesse de la réaction d'hydrogénation est gouvernée par plusieurs critères, tels que la diffusion des réactifs à la surface du catalyseur (limitations diffusionnelles externes), la diffusion des réactifs dans la porosité du support vers les sites actifs (limitations diffusionnelles internes) et les propriétés intrinsèques de la phase active telles que la taille des particules métalliques et la répartition de la phase active au sein du support.

En ce qui concerne la taille des particules métalliques, il est généralement admis que le catalyseur est d'autant plus actif que la taille des particules métalliques est petite. De plus, il est important d'obtenir une répartition en taille des particules centrée sur la valeur optimale ainsi qu'une répartition étroite autour de cette valeur.

En ce qui concerne les limitations diffusionnelles internes, il est important que la distribution poreuse des macropores et mésopores soit adaptée à la réaction souhaitée afin d'assurer la diffusion des réactifs dans la porosité du support vers les sites actifs ainsi que la diffusion des produits formés vers l'extérieur.

De nombreux développements portent ainsi sur l'optimisation de la distribution poreuse du catalyseur par l'optimisation du support du catalyseur.

Le document WO2011/080515 décrit un catalyseur d'hydrogénation à base de nickel supporté sur alumine ayant une teneur en nickel supérieure à 35% poids, ledit catalyseur ayant une grande dispersion du nickel (0) sur la surface d'une alumine à porosité très ouverte et à surface spécifique élevée. La distribution poreuse du support est bimodale : au moins 30 % du volume poreux total est constitué par des pores ayant un diamètre compris entre 5 et 20 nm, et au moins 20 % du volume poreux total est constitué de pores ayant un diamètre compris entre 100 et 700 nm avec un volume poreux total du support d'au moins 1,0 mL/g. La surface de nickel doit être supérieure ou égale à 110 m² par gramme de nickel.

Le document US 6 673 743 décrit un catalyseur à base d'alumine ayant une teneur en nickel entre 5 et 75 % poids avec une surface de nickel supérieure à 80 m² par gramme de nickel et un diamètre médian supérieure à 10 nm, ledit catalyseur étant sous forme de particules ayant une diamètre D[3,2] entre 1 et 20 µm, c'est-à-dire il est sous forme de poudre.

Le document US 5 478 791 décrit un catalyseur à base d'alumine ayant une teneur en nickel entre 10 et 60 % poids, les particules de nickel ayant un diamètre entre 15 et 50 nm. Le volume total du catalyseur est compris entre 0,3 et 0,75 g/L et entre 15 et 75 % du volume poreux total se trouve dans des pores ayant un diamètre supérieur à 100 nm. Le catalyseur présente aussi des micropores.

Enfin, le document US 4 920 089 décrit un catalyseur à base d'alumine ayant une teneur en nickel entre 5 et 40 % poids avec une surface de nickel comprise entre 80 et 300 m² par gramme de nickel. L'alumine présente un diffractogramme DRX particulier. La distribution de pores du catalyseur est comprise entre 3,5 et 30 nm, et de préférence comprise entre 4 et 20 nm.

Le document EP0885844 divulgue un catalyseur d'hydroconversion de charges d'hydrocarbures présentant une distribution poreuse monomodale avec essentiellement des mésopores de tailles comprise entre 8 et 25 nm, et un très faible volume macroporeux.

Dans ce contexte, un des objectifs de la présente invention est de proposer un catalyseur supporté à phase active de nickel ayant des performances en hydrogénation en terme d'activité au moins aussi bonnes que les catalyseurs connus de l'état de l'art.

Plus particulièrement, l'invention concerne un catalyseur supporté comprenant un support oxyde majoritairement aluminique calciné et une phase active comprenant du nickel, la teneur en nickel, mesurée par fluorescence X, étant comprise entre 5 et 65 % poids dudit élément par rapport à la masse totale du catalyseur, ladite phase active ne comprenant pas de métal du groupe VIB, ledit support a une teneur en alumine calcinée supérieure ou égale à 90% poids par rapport au poids total de ladite matrice, éventuellement complétée par de la silice et/ou du phosphore à une teneur totale d'au plus 10% poids en équivalent Si02 et/ou P205, par rapport au poids total de ladite matrice, les particules de nickel, déterminé par diffraction des rayons X, ayant un diamètre inférieur ou égal à 20 nm, ledit catalyseur ayant un diamètre médian mésoporeux, tel que mesuré par intrusion au porosimètre à mercure, compris entre 18 et 25 nm, un volume mésoporeux mesuré par porosimétrie au mercure supérieur ou égal à 0,45 mL/g, un volume poreux total mesuré par porosimétrie au mercure supérieur ou égal à 0,45 mL/g, un volume macroporeux inférieur à 5 % du volume poreux total, ledit volume mésoporeux, le volume poreux total et le volume macroporeux étant mesurés par porosité au mercure selon la norme ASTM D4284-83, ledit catalyseur étant sous forme de grains ayant un diamètre moyen compris entre 0,5 et 10 mm.

La demanderesse a découvert qu'un catalyseur préparé par imprégnation de la phase active sur une alumine résultante de la calcination d'un gel d'alumine particulier préparé selon le procédé de préparation décrit ci-après, permet l'obtention d'un catalyseur qui présente une distribution poreuse ainsi qu'une taille de particules de nickel particulièrement adaptées aux réactions d'hydrogénation, notamment aux réactions d'hydrogénation sélective de molécules polyinsaturées telles que les dioléfines, les acétyléniques ou les alcénylaromatiques, ou aux réactions d'hydrogénation des aromatiques.

En effet, la distribution poreuse résultante du procédé de préparation du support oxyde aluminique calciné issu d'un gel d'alumine spécifique, permet de fournir une porosité particulièrement adaptée à favoriser la diffusion des réactifs dans le milieu poreux puis leur réaction avec la phase active. Sans être lié à aucune théorie, il semble que les propriétés texturales particulières du catalyseur selon l'invention, notamment une porosité monomodale avec la présence de mésopores de taille contrôlée permet d'obtenir un catalyseur ayant des performances en hydrogénation en terme d'activité au moins aussi bonnes que les catalyseurs connus de l'état de l'art. Le catalyseur selon l'invention se distingue par un fort volume mésoporeux à diamètre médian des mésopores élevé couplé à un volume macroporeux de très faible valeur, voire absent. En effet, il est bien connu que bien que la présence d'un volume macroporeux puisse diminuer les limitations diffusionnelles internes, elle fragilise en même temps la résistance mécanique du catalyseur. Il est donc important de limiter le pourcentage du volume macroporeux par rapport au volume poreux total afin d'obtenir un catalyseur ayant les performances catalytiques recherchées et une résistance mécanique suffisante. De plus, la présence d'un fort volume poreux total du catalyseur selon l'invention permet d'imprégner une forte teneur en phase active en une seule passe.

Selon une variante, le volume mésoporeux du catalyseur est compris entre 0,55 mL/g et 0,95 mL/g.

Selon une variante, le volume macroporeux du catalyseur est inférieur à 3,5 % du volume poreux total.

Selon une variante, la teneur en nickel est comprise entre 10 et 34 % poids dudit élément par rapport à la masse totale du catalyseur.

Selon une variante, le catalyseur ne contient pas de pores entre 2 et 7 nm.

Selon une variante, le catalyseur ne contient pas de micropores.

L'invention concerne également le procédé de préparation dudit catalyseur. L'invention concerne aussi l'utilisation du catalyseur dans un procédé d'hydrogénation dans lequel le catalyseur selon l'invention est mis en contact en présence d'hydrogène avec une charge d'hydrocarbures contenant des molécules polyinsaturés et/ou aromatiques de manière à obtenir un effluent au moins partiellement hydrogéné.

### Description détaillée

### Le catalyseur selon l'invention

Le catalyseur selon l'invention se présente sous la forme d'un catalyseur supporté comprenant un support oxyde majoritairement aluminique calciné et une phase active comprenant du nickel. Les caractéristiques du gel d'alumine ayant conduit à l'obtention de l'alumine contenue majoritairement dans ledit support, ainsi que les propriétés texturales obtenues avec la phase active confèrent au catalyseur selon l'invention ses propriétés spécifiques.

Plus particulièrement, l'invention concerne un catalyseur supporté comprenant un support oxyde majoritairement aluminique calciné et une phase active comprenant du nickel, la teneur en nickel, mesurée par fluorescence X, étant comprise entre 5 et 65 % poids dudit élément par rapport à la masse totale du catalyseur, ladite phase active ne comprenant pas de métal du groupe VIB, ledit support a une teneur en alumine calcinée supérieure ou égale à 90% poids par rapport au poids total de ladite matrice, éventuellement complétée par de la silice et/ou du phosphore à une teneur totale d'au plus 10% poids en équivalent Si02 et/ou P205, par rapport au poids total de ladite matrice, les particules de nickel, déterminé par diffraction des rayons X, ayant un diamètre inférieur ou égal à 20 nm, ledit catalyseur ayant un diamètre médian mésoporeux, tel que mesuré par intrusion au porosimètre à mercure, compris entre 18 et 25 nm, un volume mésoporeux mesuré par porosimétrie au mercure supérieur ou égal à 0,45 mL/g, un volume poreux total mesuré par porosimétrie au mercure supérieur ou égal à 0,45 mL/g, un volume macroporeux inférieur à 5 % du volume poreux total, ledit volume mésoporeux, le volume poreux total et le volume macroporeux étant mesurés par porosité au mercure selon la norme ASTM D4284-83, ledit catalyseur étant sous forme de grains ayant un diamètre moyen compris entre 0,5 et 10 mm.

Le catalyseur selon l'invention et le support utilisé pour la préparation du catalyseur selon l'invention présentent des propriétés texturales particulières, notamment une distribution poreuse spécifique, où les volumes macroporeux et mésoporeux sont mesurés par intrusion de mercure et le volume microporeux est mesuré par adsorption d'azote.

Par « macropores », on entend des pores dont l'ouverture est supérieure à 50 nm. Par « mésopores », on entend des pores dont l'ouverture est comprise entre 2 nm et 50 nm, bornes incluses.

Par « micropores », on entend des pores dont l'ouverture est inférieure à 2 nm.

On entend par volume poreux total du catalyseur ou du support utilisé pour la préparation du catalyseur selon l'invention le volume mesuré par intrusion au porosimètre à mercure selon la norme ASTM D4284-83 à une pression maximale de 4000 bar (400 MPa), utilisant une tension de surface de 484 dyne/cm et un angle de contact de 140°. L'angle de mouillage a été pris égal à 140° en suivant les recommandations de l'ouvrage « Techniques de l'ingénieur, traité analyse et caractérisation », pages 1050-1055, écrit par Jean Charpin et Bernard Rasneur.

Afin d'obtenir une meilleure précision, la valeur du volume poreux total correspond à la valeur du volume poreux total mesuré par intrusion au porosimètre à mercure mesurée sur l'échantillon moins la valeur du volume poreux total mesuré par intrusion au porosimètre à mercure mesurée sur le même échantillon pour une pression correspondant à 30 psi (environ 0,2 MPa).

Le volume des macropores et des mésopores est mesuré par porosimétrie par intrusion de mercure selon la norme ASTM D4284-83 à une pression maximale de 4000 bar (400 MPa), utilisant une tension de surface de 484 dyne/cm et un angle de contact de 140°. On fixe à 0,2 MPa la valeur à partir de laquelle le mercure remplit tous les vides intergranulaires, et on considère qu'au-delà le mercure pénètre dans les pores de l'échantillon.

Le volume macroporeux du catalyseur ou du support utilisé pour la préparation du catalyseur selon l'invention est défini comme étant le volume cumulé de mercure introduit à une pression comprise entre 0,2 MPa et 30 MPa, correspondant au volume contenu dans les pores de diamètre apparent supérieur à 50 nm.

Le volume mésoporeux du catalyseur ou du support utilisé pour la préparation du catalyseur selon l'invention est défini comme étant le volume cumulé de mercure introduit à une pression comprise entre 30 MPa et 400 MPa, correspondant au volume contenu dans les pores de diamètre apparent compris entre 2 et 50 nm.

Le volume des micropores est mesuré par porosimétrie à l'azote. L'analyse quantitative de la microporosité est effectuée à partir de la méthode "t" (méthode de Lippens-De Boer, 1965) qui correspond à une transformée de l'isotherme d'adsorption de départ comme décrit dans l'ouvrage « Adsorption by powders and porous solids. Principles, methodology and applications » écrit par F. Rouquérol, J. Rouquérol et K. Sing, Academic Press, 1999.

On définit également le diamètre médian mésoporeux comme étant le diamètre tel que tous les pores, parmi l'ensemble des pores constituant le volume mésoporeux, de taille inférieure à ce diamètre constituent 50% du volume mésoporeux total déterminé par intrusion au porosimètre à mercure.

On définit également le diamètre médian macroporeux comme étant le diamètre tel que tous les pores, parmi l'ensemble des pores constituant le volume macroporeux, de taille inférieure à ce diamètre constituent 50% du volume macroporeux total déterminé par intrusion au porosimètre à mercure.

On entend par la surface spécifique du catalyseur ou du support utilisé pour la préparation du catalyseur selon l'invention, la surface spécifique B.E.T. déterminée par adsorption d'azote conformément à la norme ASTM D 3663-78 établie à partir de la méthode BRUNAUER-EMMETT-TELLER décrite dans le périodique « The Journal of American Society », 60, 309, (1938).

Dans la suite, les groupes d'éléments chimiques sont donnés selon la classification CAS (CRC Handbook of Chemistry and Physics, éditeur CRC press, rédacteur en chef D.R. Lide, 81ème édition, 2000-2001). Par exemple, le groupe VIII selon la classification CAS correspond aux métaux des colonnes 8, 9 et 10 selon la nouvelle classification IUPAC.

### Caractéristiques du support selon l'invention

Le support du catalyseur selon l'invention comprend de manière majoritaire un oxyde poreux aluminique calciné.

Ledit support a une teneur en alumine calcinée supérieure ou égale à 90% poids par rapport au poids total dudit support, éventuellement complétée par de la silice et/ou du phosphore à une teneur totale d'au plus 10% poids en équivalent SiO₂ et/ou P₂O₅, de préférence inférieure à 5% poids, et de manière très préférée inférieure à 2% poids par rapport au poids total de ladite matrice. La silice et/ou le phosphore peuvent être introduits par toute technique connue de l'homme du métier, lors de la synthèse du gel d'alumine ou par imprégnation du support utilisé pour la préparation du catalyseur selon l'invention.

De manière encore plus préférée, le support oxyde poreux majoritairement aluminique calciné est constitué d'alumine.

De manière préférée, l'alumine présente dans ledit support est une alumine de transition telle qu'une alumine gamma, delta, thêta, chi, rho ou êta, seule ou en mélange. De manière plus préférée, l'alumine est une alumine de transition gamma, delta ou thêta, seule ou en mélange.

Les caractéristiques du support suivantes correspondent aux caractéristiques du support utilisé pour la préparation du catalyseur selon l'invention avant imprégnation de la phase active.

Le support utilisé pour la préparation du catalyseur selon l'invention présente un volume poreux total supérieur ou égal à 0,68 mL/g, de préférence supérieur ou égal à 0,70 mL/g, et de manière particulièrement préférée compris entre 0,70 et 1,0 mL/g.

Le support utilisé pour la préparation du catalyseur selon l'invention présente avantageusement un volume macroporeux inférieur à 5 % du volume poreux total du support, de préférence inférieur à 3,5 % du volume poreux total du support. Selon une variante, le support utilisé pour la préparation du catalyseur selon l'invention présente un volume macroporeux inférieur à 0,05 mL/g.

Le volume mésoporeux du support utilisé pour la préparation du catalyseur selon l'invention est supérieur ou égal à 0,68 mL/g, de préférence supérieur ou égal à 0,70 mL/g, et de manière particulièrement préférée compris entre 0,70 et 1,0 mL/g.

Le support utilisé pour la préparation du catalyseur selon l'invention ne présente pas de pores de diamètre compris entre 2 et 7 nm.

Le diamètre médian mésoporeux du support utilisé pour la préparation du catalyseur selon l'invention est supérieur ou égal à 16 nm, de préférence supérieur ou égal à 18 nm, et de manière particulièrement préférée compris entre 20 et 25 nm.

Lorsque des macropores sont présents, le diamètre médian macroporeux du support est avantageusement compris entre 60 et 200 nm, de préférence entre 60 et 120 nm.

Le support utilisé pour la préparation du catalyseur selon l'invention présente une surface spécifique B.E.T. d'au moins 40 m²/g, de préférence d'au moins 50 m²/g, et de manière encore plus préférée comprise entre 60 et 400 m²/g.

Lorsque qu'on souhaite utiliser le catalyseur selon l'invention dans une réaction d'hydrogénation sélective de molécules polyinsaturées telles que les dioléfines, les acétyléniques ou les alcénylaromatiques, le support utilisé pour la préparation du catalyseur selon l'invention présente avantageusement une surface spécifique B.E.T. comprise entre 60 et 230 m²/g.

Lorsque qu'on souhaite utiliser le catalyseur selon l'invention dans une réaction d'hydrogénation d'aromatiques, le support utilisé pour la préparation du catalyseur selon l'invention présente avantageusement une surface spécifique B.E.T. comprise entre 130 et 400 m²/g.

De préférence, le support utilisé pour la préparation du catalyseur selon l'invention présente une faible microporosité, de manière très préférée il ne présente aucune microporosité.

### Caractéristiques du catalyseur

Le catalyseur fini, c'est-à-dire avec la phase active déposée sur le support par toute méthode connue de l'Homme du métier, comme cela est décrit ci-après, présente en conséquence les propriétés texturales à suivre.

Le catalyseur selon l'invention présente un volume poreux total supérieur ou égal à 0,45 mL/g, de préférence supérieur ou égal à 0,48 mL/g, et de manière particulièrement préférée compris entre 0,55 et 0,95 mL/g.

Le catalyseur selon l'invention présente avantageusement un volume macroporeux inférieur à 5 % du volume poreux total du catalyseur, de préférence inférieur à 3,5 % du volume poreux total du catalyseur. Selon une variante, le catalyseur selon l'invention présente un volume macroporeux inférieur à 0,05 mL/g.

Le volume mésoporeux du catalyseur est supérieur ou égal à 0,45 mL/g, de préférence supérieur ou égal à 0,48 mL/g, et de manière particulièrement préférée compris entre 0,55 et 0,95 mL/g.

Le catalyseur selon l'invention ne présente pas de pores de diamètre compris entre 2 et 7 nm.

Le diamètre médian mésoporeux du catalyseur est compris entre 18 et 25 nm.

Lorsque des macropores sont présents, le diamètre médian macroporeux du catalyseur est avantageusement compris entre 60 et 200 nm, de préférence entre 60 et 120 nm.

Le catalyseur selon la présente invention présente une surface spécifique B.E.T. d'au moins 40 m²/g, de préférence d'au moins 50 m²/g, et de manière encore plus préférée comprise entre 55 et 250 m²/g.

Lorsque qu'on souhaite utiliser le catalyseur selon l'invention dans une réaction d'hydrogénation sélective de molécules polyinsaturées telles que les dioléfines, les acétyléniques ou les alcénylaromatiques, le catalyseur selon l'invention présente avantageusement une surface spécifique B.E.T. comprise entre 55 et 170 m²/g. Lorsque qu'on souhaite utiliser le catalyseur selon l'invention dans une réaction d'hydrogénation d'aromatiques, le catalyseur selon l'invention présente avantageusement une surface spécifique B.E.T. comprise entre 90 et 250 m²/g.

De préférence, le catalyseur présente une faible microporosité, de manière très préférée il ne présente aucune microporosité.

La teneur en nickel est comprise entre 5 et 65% poids dudit élément par rapport à la masse totale du catalyseur, de préférence comprise entre 8 et 55% poids, de manière encore plus préférée comprise entre 10 et 40% poids, et de manière particulièrement préférée comprise entre 10 et 34% poids. La teneur en Ni est mesurée par fluorescence X.

Lorsque qu'on souhaite utiliser le catalyseur selon l'invention dans une réaction d'hydrogénation sélective de molécules polyinsaturées telles que les dioléfines, les acétyléniques ou les alcénylaromatiques, la teneur en nickel est avantageusement comprise entre 5 et 25% poids, de préférence comprise entre 8 et 25% poids, et plus préférentiellement comprise entre 10 et 23% poids dudit élément par rapport à la masse totale du catalyseur.

Lorsque qu'on souhaite utiliser le catalyseur selon l'invention dans une réaction d'hydrogénation d'aromatiques, la teneur en nickel est avantageusement comprise entre 15 et 65% poids, de préférence comprise entre 18 et 55% poids, et plus préférentiellement comprise entre 19 et 34% poids dudit élément par rapport à la masse totale du catalyseur.

La taille des particules de nickel dans le catalyseur selon l'invention est inférieure à 20 nm, de préférence comprise entre 1,5 et 18 nm. On comprend par « taille des particules de nickel » le diamètre des cristallites de nickel sous forme oxyde. Le diamètre des cristallites de nickel sous forme oxyde est déterminé par diffraction des rayons X, à partir de la largeur de la raie de diffraction située à l'angle 2thêta=43° (c'est-à-dire selon la direction cristallographique [200]) à l'aide de la relation de Scherrer. Cette méthode, utilisée en diffraction des rayons X sur des poudres ou échantillons polycristallins qui relie la largeur à mi-hauteur des pics de diffraction à la taille des particules, est décrite en détail dans la référence : Appl. Cryst. (1978), 11, 102-113 « Scherrer after sixty years: A survey and some new results in the détermination of crystallite size», J. I. Langford and A. J. C. Wilson.

La phase active du catalyseur peut comprendre en outre au moins un métal additionnel choisi parmi les métaux du groupe VIII, les métaux du groupe IB et/ou de l'étain. De manière préférée, le métal additionnel du groupe VIII est choisi parmi le platine, le ruthénium et le rhodium, ainsi que le palladium. Avantageusement, le métal additionnel du groupe IB est choisi parmi le cuivre, l'or et l'argent. Le(s)dit(s) métal(ux) additionnel(s) du groupe VIII et/ou du groupe IB est(sont) préférentiellement présent(s) à une teneur représentant de 0,01 à 20% poids de la masse du catalyseur, de préférence de 0,05 à 10% poids de la masse du catalyseur et de manière encore plus préférée de 0,05 à 5% poids de la masse dudit catalyseur. L'étain est préférentiellement présent à une teneur représentant de 0,02 à 15% poids de la masse du catalyseur, de telle sorte que le ratio molaire Sn/Ni soit compris entre 0,01 et 0,2, de préférence entre 0,025 à 0,055, et de manière encore plus préférée entre 0,03 à 0,05.

La phase active du catalyseur ne comprend pas de métal du groupe VIB. Il ne comprend notamment pas de molybdène ou de tungstène.

Ledit catalyseur selon l'invention est sous forme de grains ayant un diamètre moyen compris entre 0,5 et 10 mm. Les grains peuvent avoir toutes les formes connues de l'Homme du métier, par exemple la forme de billes (ayant de préférence un diamètre compris entre 1 et 6 mm), d'extrudés, de tablettes, de cylindres creux. De préférence, le catalyseur (et le support utilisé pour la préparation du catalyseur) sont sous forme d'extrudés de diamètre moyen compris entre 0,5 et 10 mm, de préférence entre 0,8 et 3,2 mm et de manière très préférée entre 1,0 et 2,5 mm et de longueur moyenne comprise entre 0,5 et 20 mm. On entend par « diamètre moyen » des extrudés le diamètre moyen du cercle circonscrit à la section droite de ces extrudés. Le catalyseur peut être avantageusement présenté sous la forme d'extrudés cylindriques, multilobés, trilobés ou quadrilobés. De préférence sa forme sera trilobée ou quadrilobée. La forme des lobes pourra être ajustée selon toutes les méthodes connues de l'art antérieur.

### Procédé de préparation

La présente invention a également pour objet un procédé de préparation dudit catalyseur selon l'invention.

Le catalyseur selon l'invention est préparé à partir d'un gel d'alumine spécifique. La distribution poreuse particulière observée dans le catalyseur est notamment due au procédé de préparation à partir du gel d'alumine spécifique.

Le procédé de préparation du gel d'alumine comprend une première étape de précipitation, une étape de chauffage, une deuxième étape de précipitation et une étape de filtration. Le gel est ensuite soumis à une étape de séchage afin d'obtenir une poudre. La poudre est ensuite mise en forme puis soumise à un traitement thermique afin d'obtenir un support oxyde poreux aluminique calciné. Le support oxyde poreux aluminique calciné est ensuite imprégné avec une solution comprenant le ou les sel(s) du ou des précurseur(s) de la phase active, puis séché pour obtenir un catalyseur séché. Puis le catalyseur séché est éventuellement soumis à un traitement thermique, puis généralement réduit et soumis à un traitement de passivation.

Plus particulièrement, le procédé de préparation du catalyseur selon l'invention comprend les étapes suivantes :
a) une première étape de précipitation, en milieu réactionnel aqueux, d'au moins un précurseur basique choisi parmi l'aluminate de sodium, l'aluminate de potassium, l'ammoniaque, l'hydroxyde de sodium et l'hydroxyde de potassium et d'au moins un précurseur acide choisi parmi le sulfate d'aluminium, le chlorure d'aluminium, le nitrate d'aluminium, l'acide sulfurique, l'acide chlorhydrique et l'acide nitrique, dans lequel au moins un des précurseurs basique ou acide comprend de l'aluminium, le débit relatif des précurseurs acide et basique est choisi de manière à obtenir un pH du milieu réactionnel compris entre 8,5 et 10,5 et le débit du ou des précurseurs acide et basique contenant de l'aluminium est réglé de manière à obtenir un taux d'avancement de la première étape compris entre 5 et 13%, le taux d'avancement étant défini comme étant la proportion d'alumine formée en équivalent Al₂O₃ lors de ladite première étape de précipitation par rapport à la quantité totale d'alumine formée en équivalent Al₂O₃ à l'issue de l'étape c) du procédé de préparation, ladite étape opérant à une température comprise entre 20 et 90°C et pendant une durée comprise entre 2 et 30 minutes,
b) une étape de chauffage de la suspension obtenue à l'étape a) à une température comprise entre 40 et 90°C pendant une durée comprise entre 7 et 45 minutes pour obtenir un gel d'alumine,
c) une deuxième étape de précipitation de la suspension obtenue à l'issue de l'étape de chauffage b) par ajout dans la suspension d'au moins un précurseur basique choisi parmi l'aluminate de sodium, l'aluminate de potassium, l'ammoniaque, l'hydroxyde de sodium et l'hydroxyde de potassium et d'au moins un précurseur acide choisi parmi le sulfate d'aluminium, le chlorure d'aluminium, le nitrate d'aluminium, l'acide sulfurique, l'acide chlorhydrique et l'acide nitrique, dans lequel au moins un des précurseurs basique ou acide comprend de l'aluminium, le débit relatif des précurseurs acide et basique est choisi de manière à obtenir un pH du milieu réactionnel compris entre 8,5 et 10,5 et le débit du ou des précurseurs acide et basique contenant de l'aluminium est réglé de manière à obtenir un taux d'avancement de la deuxième étape compris entre 87 et 95%, le taux d'avancement étant défini comme étant la proportion d'alumine formée en équivalent Al₂O₃ lors de ladite deuxième étape de précipitation par rapport à la quantité totale d'alumine formée en équivalent Al₂O₃ à l'issue de l'étape c) du procédé de préparation, ladite étape opérant à une température comprise entre 40 et 90°C et pendant une durée comprise entre 2 et 50 minutes,
d) une étape de filtration de la suspension obtenue à l'issue de l'étape c) de deuxième précipitation pour obtenir un gel d'alumine,
e) une étape de séchage dudit gel d'alumine obtenu à l'étape d) pour obtenir une poudre,
f) une étape de mise en forme de la poudre obtenue à l'issue de l'étape e) pour obtenir un matériau cru,
g) une étape de traitement thermique du matériau cru obtenu à l'issue de l'étape f) à une température comprise entre 500 et 1000°C, en présence ou non d'un flux d'air contenant jusqu'à 60% en volume d'eau, pour obtenir un support oxyde aluminique calciné,
h) une étape d'imprégnation dudit support avec une solution comprenant le ou les sel(s) du ou des précurseur(s) de la phase active à base de nickel,
i) une étape de séchage dudit support imprégné à une température comprise entre 15 et inférieure à 250°C, de manière à obtenir un catalyseur séché,
j) éventuellement un traitement thermique dudit catalyseur séché à une température comprise entre 250 et 1000°C en présence ou non d'eau.

### Etape a) Première précipitation

Cette étape consiste en la mise en contact, en milieu réactionnel aqueux, d'au moins un précurseur basique choisi parmi l'aluminate de sodium, l'aluminate de potassium, l'ammoniaque, l'hydroxyde de sodium et l'hydroxyde de potassium et d'au moins un précurseur acide choisi parmi le sulfate d'aluminium, le chlorure d'aluminium, le nitrate d'aluminium, l'acide sulfurique, l'acide chlorhydrique, et l'acide nitrique, dans laquelle au moins l'un des précurseurs basique ou acide comprend de l'aluminium, le débit relatif des précurseurs acide et basique est choisi de manière à obtenir un pH du milieu réactionnel compris entre 8,5 et 10,5 et le débit du ou des précurseurs acide et basique contenant de l'aluminium est réglé de manière à obtenir un taux d'avancement de la première étape compris entre 5 et 13%, le taux d'avancement étant défini comme étant la proportion d'alumine formée en équivalent Al₂O₃ lors de ladite première étape de précipitation par rapport à la quantité totale d'alumine formée en équivalent Al₂O₃ à l'issue de l'étape c) du procédé de préparation, ladite étape opérant à une température comprise entre 20 et 90°C, et pendant une durée comprise entre 2 et 30 minutes.

Le mélange dans le milieu réactionnel aqueux d'au moins un précurseur basique et d'au moins un précurseur acide nécessite qu'au moins un des précurseurs acide ou basique comprenne de l'aluminium. Il est également possible qu'au moins deux des précurseurs basique et acide comprennent de l'aluminium.

Les précurseurs basiques comprenant de l'aluminium sont l'aluminate de sodium et l'aluminate de potassium. Le précurseur basique préféré est l'aluminate de sodium. Les précurseurs acides comprenant de l'aluminium sont le sulfate d'aluminium, le chlorure d'aluminium et le nitrate d'aluminium. Le précurseur acide préféré est le sulfate d'aluminium.

Conformément à l'invention, les précurseurs acides d'alumine et les précurseurs basiques d'alumine peuvent être utilisés seuls ou en mélange dans l'étape de précipitation.

De préférence, le ou les précurseur(s) basique(s) et acide(s) sont ajoutés dans ladite première étape de précipitation a) en solution aqueuse. De préférence, le milieu réactionnel aqueux est de l'eau. De préférence, ladite étape a) opère sous agitation. De préférence, ladite étape a) est réalisée en l'absence d'additif organique.

Conformément à l'invention, le débit relatif des précurseurs acide et basique qu'ils contiennent de l'aluminium ou pas, est choisi de manière à obtenir un pH du milieu réactionnel compris entre 8,5 et 10,5, de préférence compris entre 8,5 et 10, et de manière très préférée compris entre 8,7 et 9,9.

Dans le cas préféré où les précurseurs basique et acide sont respectivement l'aluminate de sodium et le sulfate d'aluminium, le ratio massique dudit précurseur basique sur ledit précurseur acide est avantageusement compris entre 1,60 et 2,05. Pour les autres précurseurs basique et acide, qu'ils contiennent de l'aluminium ou pas, les ratios massiques base/acide sont établis par une courbe de neutralisation de la base par l'acide. Une telle courbe est obtenue aisément par l'Homme du métier.

La première étape a) de précipitation est réalisée à une température comprise entre 20 et 90°C, de manière préférée entre 20 et 70°C, et de manière plus préférée entre 30 et 50°C.

La première étape a) de précipitation est réalisée pendant une durée comprise entre 2 et 30 minutes, de préférence comprise entre 5 et 20 minutes, et de manière particulièrement préférée comprise entre 5 à 15 minutes.

Selon l'invention, le taux d'avancement de ladite première étape de précipitation a) est compris entre 5 et 13%, de préférence entre 6 et 12 %, et de préférence entre 7 et 11%. Le taux d'avancement pour chacune des étapes de précipitation est défini comme étant la proportion d'alumine formée en équivalent Al₂O₃ lors de ladite première ou deuxième étape de précipitation par rapport à la quantité totale d'alumine formée en équivalent Al₂O₃ à l'issue des deux étapes de précipitation et plus généralement à l'issue des étapes de préparation du gel d'alumine et notamment à l'issue de l'étape c) du procédé de préparation selon l'invention.

Les précurseurs acide et basique contenant de l'aluminium sont donc introduits dans des quantités permettant d'obtenir une suspension contenant la quantité désirée d'alumine, en fonction de la concentration finale en alumine à atteindre.

### Étape b) Chauffage

Conformément à l'invention, ledit procédé de préparation comprend une étape b) de chauffage de la suspension obtenue à l'étape a) à une température comprise entre 40 et 90°C pendant une durée comprise entre 7 et 45 minutes pour obtenir un gel d'alumine.

Ladite étape de chauffage de la suspension obtenue à l'issue de l'étape a), mise en œuvre entre ladite première étape de précipitation a) et la deuxième étape de précipitation c) opère à une température comprise entre 40 et 90°C, de préférence entre 40 et 80°C, de manière préférée entre 40 et 70°C.

Ladite étape de chauffage est mise en œuvre pendant une durée comprise entre 7 et 45 minutes, et de préférence entre 7 et 35 minutes.

Ladite étape de chauffage est avantageusement mise en œuvre selon toutes les méthodes de chauffage connues de l'Homme du métier.

### Étape c) Deuxième précipitation

Selon l'invention, ledit procédé de préparation comprend une deuxième étape de précipitation de la suspension chauffée obtenue à l'issue de l'étape b) de chauffage, ladite deuxième étape opérant par ajout dans ladite suspension d'une solution aqueuse d'au moins un précurseur basique choisi parmi l'aluminate de sodium, l'aluminate de potassium, l'ammoniaque, l'hydroxyde de sodium et l'hydroxyde de potassium et d'au moins un précurseur acide choisi parmi le sulfate d'aluminium, le chlorure d'aluminium, le nitrate d'aluminium, l'acide sulfurique, l'acide chlorhydrique, et l'acide nitrique, dans laquelle au moins l'un des précurseurs basique ou acide comprend de l'aluminium, le débit relatif des précurseurs acide et basique est choisi de manière à obtenir un pH du milieu réactionnel compris entre 8,5 et 10,5 et le débit du ou des précurseurs acide et basique contenant de l'aluminium est réglé de manière à obtenir un taux d'avancement de la deuxième étape compris entre 87 et 95%, le taux d'avancement étant défini comme étant la proportion d'alumine formée en équivalent Al₂O₃ lors de ladite deuxième étape de précipitation par rapport à la quantité totale d'alumine formée en équivalent Al₂O₃ à l'issue de l'étape c) du procédé de préparation, ladite étape opérant à une température comprise entre 40 et 90°C, et pendant une durée comprise entre 2 et 50 minutes.

De même que dans la première étape de précipitation a), l'ajout dans la suspension chauffée, d'au moins un précurseur basique et d'au moins un précurseur acide nécessite qu'au moins un des précurseurs basique ou acide comprenne de l'aluminium. Il est également possible qu'au moins deux des précurseurs basiques et acides comprennent de l'aluminium.

Les précurseurs basiques comprenant de l'aluminium sont l'aluminate de sodium et l'aluminate de potassium. Le précurseur basique préféré est l'aluminate de sodium. Les précurseurs acides comprenant de l'aluminium sont le sulfate d'aluminium, le chlorure d'aluminium et le nitrate d'aluminium. Le précurseur acide préféré est le sulfate d'aluminium.

De préférence, le ou les précurseur(s) basique(s) et acide(s) sont ajoutés dans l'étape c) en solution aqueuse. De préférence, le milieu réactionnel aqueux est de l'eau. De préférence, ladite étape c) opère sous agitation. De préférence, ladite étape c) est réalisée en l'absence d'additif organique.

De même que dans l'étape a) de précipitation, le débit relatif des précurseurs acides et basiques, qu'ils contiennent de l'aluminium ou pas, est choisi de manière à obtenir un pH du milieu réactionnel compris entre 8,5 et 10,5, de préférence compris entre 8,5 et 10, de manière encore plus préférée entre 8,7 et 9,9.

Dans le cas préféré où les précurseurs basique et acide sont respectivement l'aluminate de sodium et le sulfate d'aluminium, le ratio massique dudit précurseur basique sur ledit précurseur acide est avantageusement compris entre 1,60 et 2,05. Pour les autres précurseurs basique et acide, qu'ils contiennent de l'aluminium ou pas, les ratios massiques base/acide sont établis par une courbe de neutralisation de la base par l'acide. Une telle courbe est obtenue aisément par l'Homme du métier.

La deuxième étape de précipitation est réalisée à une température comprise entre 40 et 90°C, de préférence comprise entre 40 et 80°C, de manière préférée comprise entre 45 et 70°C, et de manière très préférée comprise entre 50 et 70°C.

La deuxième étape de précipitation est réalisée pendant une durée comprise entre 2 et 50 minutes, de préférence comprise entre 5 et 45 minutes, et de manière préférée comprise entre 7 à 40 minutes.

Les précurseurs d'aluminium sont également mélangés dans des quantités permettant d'obtenir une suspension contenant la quantité désirée d'alumine, en fonction de la concentration finale en alumine à atteindre. En particulier, ladite deuxième étape de précipitation permet l'obtention de 87 à 95% poids d'alumine par rapport à la quantité totale d'alumine formée à l'issue des deux étapes de précipitation.

De même que dans l'étape a) de précipitation, c'est le débit du ou des précurseurs acide et basique contenant de l'aluminium qui est réglé de manière à obtenir un taux d'avancement de la deuxième étape compris entre 87 et 95%, de préférence entre 88 et 94 %, de manière très préférée entre 89 et 93 %. Le taux d'avancement pour chacune des étapes de précipitation est défini comme étant la proportion d'alumine formée en équivalent Al₂O₃ lors de ladite première ou deuxième étape de précipitation par rapport à la quantité totale d'alumine formée en équivalent Al₂O₃ à l'issue des deux étapes de précipitation et plus généralement à l'issue des étapes de préparation du gel d'alumine et notamment à l'issue de l'étape c) du procédé de préparation selon l'invention.

Ainsi, en fonction de la concentration en alumine visée à l'issue des deux étapes a) et c) de précipitation, généralement comprise entre 20 et 100 g/L, de préférence entre 20 et 80 g/L, de manière préférée entre 20 et 50 g/L, les quantités d'aluminium devant être apportées par les précurseurs acide et/ou basique sont calculées et le débit des précurseurs est réglé en fonction de la concentration desdits précurseurs en aluminium ajoutés, de la quantité d'eau ajoutée dans le milieu réactionnel et du taux d'avancement requis pour chacune des étapes de précipitation.

De même que dans l'étape a) de précipitation, les débits du ou des précurseurs acide et/ou basique contenant de l'aluminium dépendent de la dimension du réacteur utilisé et ainsi de la quantité d'eau ajoutée dans le milieu réactionnel.

A titre d'exemple, si on travaille dans un réacteur de 3 L et que l'on vise 1L de suspension d'alumine de concentration finale en Al₂O₃ de 50 g/L, avec un taux d'avancement ciblé de 10% pour la première étape de précipitation, 10% de l'alumine totale doit être apportée lors de l'étape a) de précipitation. Les précurseurs d'alumine sont l'aluminate de sodium à une concentration de 155 g/L en Al₂O₃ et le sulfate d'aluminium à une concentration de 102 g/L en Al₂O₃ Le pH de précipitation de la première étape est fixé à 9,5 et la deuxième à 9. La quantité d'eau ajoutée dans le réacteur est de 620 mL.

Pour la première étape a) de précipitation opérant à 30°C et pendant 8 minutes, le débit de sulfate d'aluminium doit être de 2,1 mL/min et le débit d'aluminate de sodium est de 2,6 mL/min. Le ratio massique d'aluminate de sodium sur sulfate d'aluminium est donc de 1,91.

Pour la deuxième étape de précipitation, opérant à 70°C, pendant 30 minutes, le débit de sulfate d'aluminium doit être de 5,2 mL/min et le débit d'aluminate de sodium est de 6,3 mL/min. Le ratio massique d'aluminate de sodium sur sulfate d'aluminium est donc de 1,84.

### Étape d) Filtration

Le procédé de préparation d'alumine selon l'invention comprend également une étape de filtration de la suspension obtenue à l'issue de l'étape c) de deuxième précipitation de manière à obtenir un gel d'alumine. Ladite étape de filtration est réalisée selon les méthodes connues de l'Homme du métier.

Ladite étape de filtration est avantageusement suivie d'au moins une étape de lavage, de préférence à l'eau et de préférence d'une à trois étapes de lavage, avec une quantité d'eau égale à la quantité de précipité filtré.

La filtrabilité de la suspension obtenue à l'issue des deux étapes de précipitation est améliorée par la faible dispersibilité du gel d'alumine obtenu, ce qui permet d'améliorer la productivité du procédé selon l'invention ainsi que de permettre une extrapolation du procédé au niveau industriel. La dispersibilité est définie comme le poids de solide ou gel d'alumine peptisée que l'on ne peut pas disperser par centrifugation dans un tube en polypropylène à 3600G pendant 3 minutes.

A la fin de l'étape d) on obtient un gel d'alumine, aussi appelé boehmite, présentant un taux de dispersibilité inférieur ou égal à 15%, de préférence compris entre 5 et 15%, et de manière préférée comprise entre 6 et 14%, et de manière très préférée compris entre 7 et 13%, et de manière encore plus préférée compris entre 7 et 10% et une taille de particules de boehmite comprise entre 1 et 35 nm et de préférence comprise entre 2 à 35 nm.

Le taux de dispersibilité faible du gel ainsi préparé permet de faciliter l'étape de mise en forme dudit gel selon toutes les méthodes connues de l'Homme du métier et en particulier par malaxage extrusion, par granulation, par pastillage et par la technique dite de la goutte d'huile (égouttage).

### Etape e) Séchage du gel d'alumine

Conformément à l'invention, le gel d'alumine obtenu à l'issue de l'étape c) de deuxième précipitation, suivie d'une étape de filtration d), est séché dans une étape e) de séchage pour obtenir une poudre. Ladite étape de séchage est généralement mise en œuvre par séchage à une température comprise entre 20 et 200°C et pendant une durée comprise entre 8 et 15 heures, ou par atomisation ou par toute autre technique de séchage connue de l'Homme du métier.

Dans le cas où ladite étape e) de séchage est mise en œuvre par atomisation, le « gâteau » obtenu à l'issue de l'étape de deuxième précipitation, suivie d'une étape de filtration, est remis en suspension. Ladite suspension est ensuite pulvérisée en fines gouttelettes, dans une enceinte cylindrique verticale au contact d'un courant d'air chaud afin d'évaporer l'eau selon le principe bien connu de l'Homme du métier. La poudre obtenue est entrainée par le flux de chaleur jusqu'à un cyclone ou un filtre à manche qui va séparer l'air de la poudre.

De préférence, dans le cas où ladite étape e) de séchage est mise en œuvre par atomisation, l'atomisation est réalisée selon le protocole opératoire décrit dans la publication Asep Bayu Dani Nandiyanto, Kikuo Okuyama, Advanced Powder Technology, 22, 1-19, 2011.

### Étape f) Mise en forme

Conformément à l'invention, la poudre obtenue à l'issue de l'étape e) de séchage est mise en forme dans une étape f) pour obtenir un matériau cru. On entend par « matériau cru », le matériau mis en forme et n'ayant pas subi d'étapes de traitement thermique.

De préférence, ladite étape f) de mise en forme est réalisée selon toute technique connue de l'Homme du métier, par exemple les méthodes de mise en forme par extrusion, par pastillage, par la méthode de la goutte d'huile (égouttage) ou par granulation au plateau tournant.

De manière très préférée, ladite étape f) de mise en forme est réalisée par extrusion. On peut utiliser une extrudeuse piston au travers d'une filière ayant le diamètre souhaité, typiquement entre 0,5 et 10 mm. Les extrudés ont généralement un diamètre moyen compris entre 0,5 et 10 mm, de préférence 0,8 et 3,2 mm, et de manière très préférée entre 1,0 et 2,5 mm, et une longueur moyenne comprise entre 0,5 et 20 mm. Les extrudés peuvent être avantageusement présentés sous la forme d'extrudés cylindriques, multilobés, trilobés ou quadrilobés. De préférence la forme sera trilobée ou quadrilobée.

Tout autre élément, par exemple de la silice sous forme d'une solution ou une émulsion de précurseur silicique, peut être introduit lors de la mise en forme.

### Étape g) Traitement thermique

Conformément à l'invention, le matériau cru obtenu à l'issue de l'étape f) de mise en forme subit ensuite une étape g) de traitement thermique à une température comprise entre 500 et 1000°C, en présence ou non d'un flux d'air contenant jusqu'à 60% volume d'eau.

De préférence, ladite étape g) de traitement thermique opère à une température comprise entre 540 et 850°C. De préférence, ladite étape g) de traitement thermique opère pendant une durée comprise entre 2 et 10 heures. L'étape de traitement thermique peut être précédée d'un séchage à une température comprise entre 50°C et 200°C, selon toute technique connue de l'Homme du métier.

On entend par « traitement thermique » le traitement en température respectivement sans présence ou en présence d'eau. Dans ce dernier cas, le contact avec la vapeur d'eau peut se dérouler à pression atmosphérique (« steaming ») ou en pression autogène (autoclavage). Plusieurs cycles combinés sans présence ou avec présence d'eau peuvent être réalisés.

En cas de présence d'eau, la teneur en eau est de préférence comprise entre 150 et 900 grammes par kilogramme d'air sec, et de manière encore plus préférée, entre 250 et 650 grammes par kilogramme d'air sec.

Ladite étape g) de traitement thermique permet la transition du gel d'alumine, aussi appelé boehmite, vers une alumine calcinée. L'alumine présente une structure cristallographique du type alumine de transition gamma, delta, thêta, chi, rho ou êta, seule ou en mélange. De manière plus préférée, l'alumine est une alumine de transition gamma, delta ou thêta, seule ou en mélange. L'existence des différentes structures cristallographiques est liée aux conditions de mises en œuvre de l'étape g) de traitement thermique.

### Etape h) Imprégnation de la phase active

Selon l'étape h) du procédé selon l'invention, on imprègne le support oxyde poreux aluminique calciné avec une solution comprenant le ou les sel(s) du ou des précurseur(s) de la phase active à base de nickel.

La phase active est apportée par une ou plusieurs solutions contenant au moins du nickel.

La(les)dite(s) solution(s) peu(ven)t être aqueuse(s) ou constituée(s) d'un solvant organique ou bien d'un mélange d'eau et d'au moins un solvant organique (par exemple l'éthanol ou le toluène). De préférence, la solution est aqueuse. Le pH de cette solution pourra être modifié par l'ajout éventuel d'un acide. Selon une autre variante préférée, la solution aqueuse peut contenir de l'ammoniaque ou des ions d'ammonium NH₄⁺.

De manière préférée, ledit précurseur de nickel est introduit en solution aqueuse, par exemple sous forme de nitrate, de carbonate, d'acétate, de chlorure, d'hydroxyde, d'hydroxycarbonate, d'oxalate, de complexes formés par un polyacide ou un acide-alcool et ses sels, de complexes formés avec les acétylacétonates, ou de tout autre dérivé inorganique soluble en solution aqueuse, laquelle est mise en contact avec ledit oxyde poreux aluminique calciné. De manière préférée, on utilise avantageusement comme précurseur de nickel, le nitrate de nickel, le chlorure de nickel, l'acétate de nickel ou le hydroxycarbonate de nickel. De manière très préférée, le précurseur de nickel est le nitrate de nickel ou le hydroxycarbonate de nickel. Selon une autre variante préférée, ledit précurseur de nickel est introduit en solution ammoniacale en introduisant un sel de nickel, par exemple l'hydroxyde de nickel ou le carbonate de nickel dans une solution ammoniacale ou dans une solution de carbonate d'ammonium ou d'hydrogénocarbonate d'ammonium.

Les quantités du ou des précurseurs de nickel introduites dans la solution sont choisies de telle manière que la teneur totale en nickel est comprise entre 5 et 65 % poids, de préférence comprise entre 8 et 55 % poids, de manière préférée comprise entre 10 et 40 % poids, et de manière particulièrement préférée comprise entre 10 et 34 % poids dudit élément par rapport à la masse totale du catalyseur. Les teneurs en nickel sont généralement adaptées à la réaction d'hydrogénation visée tel que décrit ci-dessus dans le paragraphe de la description du catalyseur.

Tout autre élément supplémentaire peut être introduit au moment de cette étape : Lorsqu'on souhaite introduire du phosphore, une solution d'acide phosphorique peut être introduite dans la solution d'imprégnation.

Lorsqu'on souhaite introduire un métal additionnel choisi parmi les métaux du groupe VIII, les métaux du groupe IB et/ou de l'étain, on utilise avantageusement comme précurseur un sel choisi parmi le nitrate, le sulfate, le chlorure ou tout autre précurseur conventionnel.

Un additif, par exemple un agent chélatant de nature organique, peut avantageusement être introduit dans la solution si l'Homme du métier le juge nécessaire.

L'imprégnation de la phase active peut être réalisée selon toutes les méthodes connues de l'Homme du métier, en particulier par imprégnation à sec. De façon préférée, le nickel et éventuellement au moins un élément supplémentaire tel qu'un métal additionnel choisi parmi les métaux du groupe VIII, les métaux du groupe IB et/ou de l'étain, du phosphore ou un additif tel qu'un agent chélatant de nature organique sont déposés par imprégnation à sec de leurs précurseurs associés sur le support oxyde selon l'invention.

Le dépôt peut se faire via une seule étape d'imprégnation à sec du support oxyde selon l'invention via l'emploi d'une solution contenant simultanément au moins un composé de nickel, et éventuellement au moins un élément supplémentaire.

Le dépôt peut aussi être avantageusement réalisé via au moins deux cycles d'imprégnation à sec. Les différents éléments peuvent ainsi être avantageusement imprégnés successivement ou bien un des éléments peut aussi être imprégné en plusieurs séquences. Une des imprégnations qui est réalisée peut notamment permettre d'introduire un composé organique en plus de la phase active du catalyseur. Dans ces cas, chaque imprégnation est avantageusement suivie d'un séchage et éventuellement d'un traitement thermique. Le séchage peut être réalisé à une température comprise entre 15 et 250°C, de préférence entre 80 et 200°C, généralement pendant une durée comprise entre 10 minutes et 24 heures. Le traitement thermique peut être réalisé à une température comprise entre 200 et 1000°C, préférentiellement entre 250 et 750°C, généralement pendant une durée comprise entre 15 minutes et 10 heures.

### Etape i) Séchage du support imprégné

Conformément à l'invention, le support imprégné obtenu à l'issue de l'étape h) d'imprégnation de la phase active subit une étape de séchage i) à une température comprise entre 15 et inférieure à 250°C, de préférence entre 80 et 200°C, selon toute technique connue de l'Homme du métier, pendant une durée typiquement comprise entre 10 minutes et 24 heures. On obtient un catalyseur séché.

### Etape i) Traitement thermique du catalyseur séché

Le catalyseur ainsi séché peut ensuite subir une étape complémentaire de traitement thermique j) à une température comprise entre 250 et 1000°C et de préférence entre 250 et 750°C, pendant une durée typiquement comprise entre 15 minutes et 10 heures, en présence ou non d'eau.

On entend par « traitement thermique » le traitement en température respectivement sans présence ou en présence d'eau. Dans ce dernier cas, le contact avec la vapeur d'eau peut se dérouler à pression atmosphérique (« steaming ») ou en pression autogène (autoclavage). Plusieurs cycles combinés de traitements thermiques ou hydrothermiques peuvent être réalisés. Après ce ou ces traitement(s), le précurseur de catalyseur comprend du nickel sous forme oxyde, c'est-à-dire sous forme NiO.

En cas de traitement hydrothermique, la teneur en eau est de préférence comprise entre 150 et 900 grammes par kilogramme d'air sec, et de manière encore plus préférée, entre 250 et 650 grammes par kilogramme d'air sec.

### Etape k) Réduction par un gaz réducteur

Préalablement à l'utilisation du catalyseur dans le réacteur catalytique et la mise en œuvre d'un procédé d'hydrogénation, on effectue avantageusement au moins une étape de traitement réducteur k) en présence d'un gaz réducteur après les étapes i) ou j) de manière à obtenir un catalyseur comprenant du nickel au moins partiellement sous forme métallique.

Ce traitement permet d'activer ledit catalyseur et de former des particules métalliques, en particulier du nickel à l'état zéro valent. Ledit traitement réducteur peut être réalisé *in-situ* ou *ex-situ* c'est-à-dire après ou avant le chargement du catalyseur dans le réacteur d'hydrogénation. Ladite étape k) de traitement réducteur peut être mise en œuvre sur le catalyseur ayant été soumis ou non à l'étape l) de passivation, décrite par la suite.

Le gaz réducteur est de préférence l'hydrogène. L'hydrogène peut être utilisé pur ou en mélange (par exemple un mélange hydrogène/azote, hydrogène/argon, hydrogène/méthane). Dans le cas où l'hydrogène est utilisé en mélange, toutes les proportions sont envisageables.

Ledit traitement réducteur est réalisé à une température comprise entre 120 et 500°C, de préférence entre 150 et 450°C. Lorsque le catalyseur ne subit pas de passivation, ou subit un traitement réducteur avant passivation, le traitement réducteur est effectué à une température comprise entre 350 et 500°C, de préférence entre 350 et 450°C. Lorsque le catalyseur a subi au préalable une passivation, le traitement réducteur est généralement effectué à une température comprise entre 120 et 350°C, de préférence entre 150 et 350°C.

La durée du traitement réducteur est généralement comprise entre 2 et 40 heures, de préférence entre 3 et 30 heures. La montée en température jusqu'à la température de réduction désirée est généralement lente, par exemple fixée entre 0,1 et 10°C/min, de préférence entre 0,3 et 7°C/min.

Le débit d'hydrogène, exprimé en L/heure/gramme de catalyseur est compris entre 0,1 et 100 L/heure/gramme de catalyseur, de préférence entre 0,5 et 10 L/heure/gramme de catalyseur, de façon encore plus préférée entre 0,7 et 5 L/heure/gramme de catalyseur.

### Etape I) Passivation

Préalablement à sa mise en œuvre dans le réacteur catalytique, le catalyseur selon l'invention peut éventuellement subir une étape de passivation (étape l) par un composé soufré ou oxygéné ou par le CO₂ avant ou après l'étape de traitement réducteur k). Cette étape de passivation peut être effectuée *ex-situ* ou *in-situ.* L'étape de passivation est réalisée par la mise en œuvre de méthodes connues de l'Homme du métier.

L'étape de passivation par le soufre permet d'améliorer la sélectivité des catalyseurs et d'éviter les emballements thermiques lors des démarrages de catalyseurs neufs (« run away » selon la terminologie anglo-saxonne). La passivation consiste généralement à empoisonner irréversiblement par le composé soufré les sites actifs les plus virulents du nickel qui existent sur le catalyseur neuf et donc à atténuer l'activité du catalyseur en faveur de sa sélectivité. L'étape de passivation est réalisée par la mise en œuvre de méthodes connues de l'Homme du métier et notamment, à titre d'exemple par la mise en œuvre de l'une des méthodes décrites dans les documents de brevets EP0466567, US5153163, FR2676184, WO2004/098774, EP0707890. Le composé soufré est par exemple choisi parmi les composés suivants: thiophène, thiophane, alkylmonosulfures tels que diméthylsulfure, diéthylsulfure, dipropylsulfure et propylméthylsulfure ou encore un disulfure organique de formule HO-R₁-S-S-R₂-OH tel que le di-thio-di-éthanol de formule HO-C₂H₄-S-S-C₂H₄-OH (appelé souvent DEODS). La teneur en soufre est généralement comprise entre 0,1 et 2 % poids dudit élément par rapport à la masse du catalyseur.

L'étape de passivation par un composé oxygéné ou par le CO₂ est généralement effectuée après un traitement réducteur au préalable à température élevée, généralement comprise entre 350 et 500°C, et permet de préserver la phase métallique du catalyseur en présence d'air. Un deuxième traitement réducteur à température plus basse généralement entre 120 et 350°C, est ensuite généralement effectué. Le composé oxygéné est généralement l'air ou tout autre flux contenant de l'oxygène.

### Procédé d'hydrogénation sélective

La présente invention concerne également l'utilisation du catalyseur selon l'invention dans un procédé d'hydrogénation et notamment dans un procédé d'hydrogénation sélective de composés polyinsaturées telles que les dioléfines, les acétyléniques ou les alcénylaromatiques, aussi appelés styréniques.

Les composés organiques mono-insaturés tels que par exemple l'éthylène et le propylène, sont à la source de la fabrication de polymères, de matières plastiques et d'autres produits chimiques à valeur ajoutée. Ces composés sont obtenus à partir du gaz naturel, du naphta ou du gazole qui ont été traités par des procédés de vapocraquage ou de craquage catalytique. Ces procédés sont opérés à haute température et produisent, en plus des composés mono-insaturés recherchés, des composés organiques polyinsaturés tels que l'acétylène, le propadiène et le méthylacétylène (ou propyne), le 1-2-butadiène et le 1-3-butadiène, le vinylacétylène et l'éthylacétylène, et d'autres composés polyinsaturés dont le point d'ébullition correspond à la fraction essence C5+ (essences contenant des composés hydrocarbonés ayant 5 atomes de carbone ou plus), en particulier des composés dioléfiniques ou styréniques ou indéniques. Ces composés polyinsaturés sont très réactifs et conduisent à des réactions parasites dans les unités de polymérisation. Il est donc nécessaire de les éliminer avant de valoriser ces coupes.

L'hydrogénation sélective est le principal traitement développé pour éliminer spécifiquement les composés polyinsaturés indésirables de ces charges d'hydrocarbures. Elle permet la conversion des composés polyinsaturés vers les alcènes ou aromatiques correspondants en évitant leur saturation totale et donc la formation des alcanes ou naphtènes correspondants. Dans le cas d'essences de vapocraquage utilisées comme charge, l'hydrogénation sélective permet également d'hydrogéner sélectivement les alcénylaromatiques en aromatiques en évitant l'hydrogénation des noyaux aromatiques.

La charge d'hydrocarbures traitée dans le procédé d'hydrogénation sélective a un point d'ébullition final inférieur ou égal à 250°C et contient au moins 2 atomes de carbone par molécule et comprend au moins un composé polyinsaturé. On entend par « composés polyinsaturés » des composés comportant au moins une fonction acétylénique et/ou au moins une fonction diénique et/ou au moins une fonction alcénylaromatique.

Plus particulièrement, la charge est sélectionnée dans le groupe constitué par une coupe C2 de vapocraquage, une coupe C3 de vapocraquage, une coupe C4 de vapocraquage, une coupe C5 de vapocraquage et une essence de vapocraquage encore appelée essence de pyrolyse. L'essence de vapocraquage ou essence de pyrolyse correspond à une coupe hydrocarbonée dont la température d'ébullition est généralement comprise entre 0 et 250°C, de préférence entre 10 et 220°C. Les hydrocarbures polyinsaturés à hydrogéner présents dans ladite essence de vapocraquage sont en particulier des composés dioléfiniques (butadiène, isoprène, cyclopentadiène...), des composés styréniques (styrène, alpha-méthylstyrène...) et des composés indéniques (indène...). L'essence de vapocraquage comprend généralement la coupe C5-C12 avec des traces de C3, C4, C13, C14, C15 (par exemple entre 0,1 et 3% poids pour chacune de ces coupes). Par exemple, une charge formée d'essence de pyrolyse a généralement une composition suivante: 5 à 25 % poids de paraffines, 40 à 70 % poids de composés aromatiques, 5 à 20 % poids de mono-oléfines, 5 à 40 % poids de dioléfines, 1 à 10 % poids de composés alcénylaromatiques et de 20 à 300 ppm poids de soufre, l'ensemble des composés formant 100%. De manière préférée, la charge d'hydrocarbures polyinsaturés traitée conformément au procédé d'hydrogénation sélective selon l'invention est une essence de vapocraquage.

Le procédé d'hydrogénation sélective selon l'invention vise à éliminer lesdits hydrocarbures polyinsaturés présents dans ladite charge à hydrogéner sans hydrogéner les hydrocarbures monoinsaturés. Par exemple, lorsque ladite charge est une coupe C2, le procédé d'hydrogénation sélective vise à hydrogéner sélectivement l'acétylène. Lorsque ladite charge est une coupe C3, le procédé d'hydrogénation sélective vise à hydrogéner sélectivement le propadiène et le méthylacétylène. Dans le cas d'une coupe C4, on vise à éliminer le butadiène, le vinylacétylène (VAC) et le butyle, dans le cas d'une coupe C5, on vise à éliminer les pentadiènes. Lorsque ladite charge est une essence de vapocraquage, le procédé d'hydrogénation sélective vise à hydrogéner sélectivement lesdits hydrocarbures polyinsaturés présents dans ladite charge à traiter de manière à ce que les composés dioléfiniques soient partiellement hydrogénés en mono-oléfines et que les composés styréniques et indéniques soient partiellement hydrogénés en composés aromatiques correspondants en évitant l'hydrogénation des noyaux aromatiques.

La mise en œuvre technologique du procédé d'hydrogénation sélective est par exemple réalisée par injection, en courant ascendant ou descendant, de la charge d'hydrocarbures polyinsaturés et de l'hydrogène dans au moins un réacteur à lit fixe. Ledit réacteur peut être de type isotherme ou de type adiabatique. Un réacteur adiabatique est préféré. La charge d'hydrocarbures polyinsaturés peut avantageusement être diluée par une ou plusieurs ré-injection(s) de l'effluent, issu dudit réacteur où se produit la réaction d'hydrogénation sélective, en divers points du réacteur, situés entre l'entrée et la sortie du réacteur afin de limiter le gradient de température dans le réacteur. La mise en œuvre technologique du procédé d'hydrogénation sélective selon l'invention peut également être avantageusement réalisée par l'implantation d'au moins dudit catalyseur supporté dans une colonne de distillation réactive ou dans des réacteurs - échangeurs. Le flux d'hydrogène peut être introduit en même temps que la charge à hydrogéner et/ou en un ou plusieurs points différents du réacteur.

L'hydrogénation sélective des coupes C2, C3, C4, C5 et C5+ peut être réalisée en phase gazeuse ou en phase liquide, de préférence en phase liquide pour les coupes C3, C4, C5 et C5+ . En effet, une réaction en phase liquide permet d'abaisser le coût énergétique et d'augmenter la durée de cycle du catalyseur.

D'une manière générale, l'hydrogénation sélective s'effectue à une température comprise entre 0 et 500°C, à une pression comprise entre 0,1 et 20 MPa, à un ratio molaire hydrogène/(composés polyinsaturés à hydrogéner) entre 0,1 et 10 et à une vitesse volumique horaire V.V.H. (définie comme le rapport du débit volumique de charge sur le volume du catalyseur) comprise entre 0,1 et 200 h⁻¹ pour une charge liquide, entre 100 et 15 000 h⁻¹ pour une charge gazeuse, d'une charge d'hydrocarbures contenant des composés polyinsaturés contenant au moins 2 atomes de carbone par molécule et ayant un point d'ébullition final inférieur ou égal à 250°C.

De manière préférée, on effectue un procédé d'hydrogénation sélective dans lequel la charge est une essence de vapocraquage comportant des composés polyinsaturés, le ratio molaire (hydrogène)/(composés polyinsaturés à hydrogéner) est généralement compris entre 1 et 2, la température est généralement comprise entre 40 et 200°C, de préférence entre 50 et 180°C, la vitesse volumique horaire (V.V.H.) est comprise généralement entre 0,5 et 50 h⁻¹, de préférence entre 1 et 20 h⁻¹ et la pression est généralement comprise entre 0,3 et 6,5 MPa, de préférence entre 2,0 et 3,5 MPa. Le débit d'hydrogène est ajusté afin d'en disposer en quantité suffisante pour hydrogéner théoriquement l'ensemble des composés polyinsaturés et de maintenir un excès d'hydrogène en sortie de réacteur.

### Procédé d'hydrogénation des aromatiques

La présente invention concerne également l'utilisation du catalyseur selon l'invention dans un procédé d'hydrogénation et notamment dans un procédé d'hydrogénation des aromatiques permettant de transformer les composés aromatiques de coupes pétrolières ou pétrochimiques par conversion des noyaux aromatiques en noyaux naphténiques.

La charge d'hydrocarbures traitée dans le procédé d'hydrogénation des aromatiques a un point d'ébullition final inférieur ou égal à 650°C, généralement entre 20 et 650°C, et de préférence entre 20 et 450°C, et contient au moins un composé aromatique ou polyaromatique. Comme coupe pétrolière ou pétrochimique contenant des composés aromatiques on peut par exemple citer le kérosène, le gazole léger, le gazole lourd et des distillats de craquage, tels que l'huile de recyclage FCC, le gazole d'unité de cokéfaction, des distillats d'hydrocraquage, et le reformat du reformage catalytique.

La teneur en hydrocarbures aromatiques dans une charge traitée dans le procédé d'hydrogénation est généralement comprise entre 0,1 et 80 % poids, de préférence entre 1 et 50 % poids, et de manière particulièrement préférée entre 2 et 35 % poids le pourcentage en poids étant basé sur le poids total de la charge d'hydrocarbures. Les aromatiques présents sont par exemple le benzène ou des alkylaromatiques tels que le toluène, l'éthylbenzène, l'o-xylène, le m-xylène, ou le p-xylène, ou encore des aromatiques ayant plusieurs noyaux aromatiques (polyaromatiques) tels que le naphtalène.

La teneur en soufre ou en chlore de la charge est généralement inférieure à 5000 ppm poids de soufre ou chlore respectivement, de préférence inférieure à 100 ppm poids, et de manière particulièrement préférée inférieure à 10 ppm.

La mise en œuvre technologique du procédé d'hydrogénation des aromatiques peut être effectuée telle que celle décrite dans la partie hydrogénation sélective.

L'hydrogénation des aromatiques peut être réalisée en phase gazeuse ou en phase liquide, de préférence en phase liquide. D'une manière générale, l'hydrogénation des aromatiques s'effectue à une température comprise entre 30 et 350°C, de préférence entre 50 et 325°C, à une pression comprise entre 0,1 et 20 MPa, de préférence entre 0,5 et 10 MPa, à un ratio molaire hydrogène/(composés aromatiques à hydrogéner) entre 0,1 et 10 et à une vitesse volumique horaire V.V.H. comprise entre 0,05 et 50 h⁻¹, de préférence entre 0,1 et 10 h⁻¹, d'une charge d'hydrocarbures contenant des composés aromatiques et ayant un point d'ébullition final inférieur ou égal à 650°C. Le débit d'hydrogène est ajusté afin d'en disposer en quantité suffisante pour hydrogéner théoriquement l'ensemble des composés polyinsaturés et de maintenir un excès d'hydrogène en sortie de réacteur.

La conversion des composés aromatiques ou polyaromatiques est généralement supérieure à 20% en mole, de préférence supérieure à 40% en mole, de manière plus préférée supérieure à 80% en mole, et de manière particulièrement préférée supérieure à 90 % en mole des composés aromatiques ou polyaromatiques contenus dans la charge hydrocarbonée. La conversion se calcule en divisant la différence entre les moles totales des composés aromatiques ou polyaromatiques dans la charge d'hydrocarbures et dans le produit par les moles totales des composés aromatiques ou polyaromatiques dans la charge d'hydrocarbures.

Selon une variante particulière, le catalyseur selon l'invention est utilisé dans un procédé d'hydrogénation d'une charge d'hydrocarbures contenant du benzène tel que par exemple le reformat issu d'une unité de reformage catalytique. La teneur en benzène est généralement comprise entre 0,1 et 40% poids, de préférence entre 0,5 et 35% poids, et de manière particulièrement préférée entre 2 et 30% poids, le pourcentage en poids étant basé sur le poids total de la charge d'hydrocarbures.

La teneur en soufre ou en chlore de la charge est généralement inférieure à 10 ppm poids de soufre ou chlore respectivement, et de préférence inférieure à 2 ppm poids.

L'hydrogénation de la charge contenant du benzène peut être réalisée en phase gazeuse ou en phase liquide, de préférence en phase liquide. Lorsqu'elle est réalisée en phase liquide, un solvant peut être présent. D'une manière générale, l'hydrogénation du benzène s'effectue à une température comprise entre 30 et 250°C, de préférence entre 50 et 200°C, et de manière plus préférée entre 80 et 180°C, à une pression comprise entre 0,1 et 10 MPa, de préférence entre 0,5 et 4 MPa, à un ratio molaire hydrogène/(benzène) entre 0,1 et 10 et à une vitesse volumique horaire V.V.H. comprise entre 0,05 et 50 h⁻¹, de préférence entre 0,5 et 10 h⁻¹.

La conversion du benzène est généralement supérieure à 50% en mole, de préférence supérieure à 80% en mole, de manière plus préférée supérieure à 90% en mole et de manière particulièrement préférée supérieure à 98 % en mole.

L'invention est illustrée par les exemples qui suivent.

### Exemples

### Exemple 1 : Préparation d'une solution aqueuse de précurseurs de Ni

La solution aqueuse de précurseurs de Ni (solution S) utilisée pour la préparation des catalyseurs A, B et C est préparée en dissolvant 46,1 g de nitrate de nickel (NiNO₃, fournisseur Strem Chemicals®) dans un volume de 13 mL d'eau distillée. On obtient la solution S dont la concentration en NiO est de 20,1 %pds (par rapport à la masse de la solution).

### Exemple 2 : Préparation d'un catalyseur A, selon l'invention

Le catalyseur A selon l'invention est préparé par imprégnation à sec de la solution S de précurseurs de Ni sur une alumine A1. On vise une teneur de 20 % poids en nickel par rapport à la masse totale du catalyseur.

La synthèse de l'alumine A1 selon l'invention est réalisée dans un réacteur de 5 L en sept étapes, nommées ci-dessous a) à g). La concentration des précurseurs acide et basique d'alumine est la suivante : sulfate d'aluminium Al₂(SO₄)₃ à 102 g/L en Al₂O₃ et aluminate de sodium NaAlOO à 155 g/L en Al₂O₃. On cherche à obtenir une concentration finale en alumine de 45 g/L dans la suspension obtenue à l'issue de la deuxième étape c) de précipitation.
a) Une première précipitation du sulfate d'aluminium Al₂(SO₄)₃ et de l'aluminate de sodium NaAlOO en 8 minutes à 30 °C, pH=9,1 et avec un taux d'avancement de 10 %. Ce taux d'avancement correspond à la proportion d'alumine formée en équivalent Al₂O₃ lors de cette première étape.
b) Une montée en température de 30 °C à 70 °C en 20 à 30 minutes.
c) Une deuxième précipitation du sulfate d'aluminium Al₂(SO₄)₃ et de l'aluminate de sodium NaAlOO en 30 minutes à 70 °C, pH=9,1 et avec un taux d'avancement de 90 %. Ce taux d'avancement correspond à la proportion d'alumine formée en équivalent Al₂O₃ lors de cette deuxième étape de précipitation.
d) Une filtration de la suspension obtenue à l'issue de l'étape c) par déplacement sur un outil de type Buchner fritté P4, suivie de trois lavages successifs avec 5 L d'eau distillée.
e) Un séchage du gel d'alumine pendant une nuit à 120 °C.
f) Le gel d'alumine séché issu de l'étape e) est mis en forme à l'aide d'un malaxeur de type "Brabender" avec un taux d'acide de 3 % (taux d'acide total, exprimé par rapport à l'alumine sèche), un taux de neutralisation de 200 % et des pertes au feu acide et basique respectivement de 62 % et 64 %. Puis l'extrusion est effectuée sur une extrudeuse piston à travers une filière trilobée de diamètre moyen de 2,1 mm. Après extrusion, les extrudés sont séchés une nuit à 80 °C.
g) Les extrudés obtenus à l'issue de l'étape f) sont ensuite calcinés à 750 °C sous un flux d'air de 1L/h/g d'alumine à 750°C pendant 2 heures (rampe de montée en température de 5°C/min). On obtient l'alumine A1.
Les caractéristiques de l'alumine A1 ainsi obtenue sont reportées dans le Tableau 1 ci-après. L'alumine A1 ne contient pas de pores de diamètre comprise entre 2 et 7 nm.

**Tableau 1 : Propriétés des alumines A1 (selon l'invention), et B1 et C1 (comparatifs)**

| ALUMINES | A1 | B1 | C1 |
|---|---|---|---|
| | Selon l'invention | Comparatif | Comparatif |
| Surface B.E.T. (m²/g) | 156 | 269 | 298 |
| Volume poreux total (mL/g) | 0,85 | 0,64 | 0,60 |
| Volume mésoporeux (mL/g) | 0,83 | 0,61 | 0,57 |
| Diamètre médian mésoporeux (nm) | 21 | 9,4 | 17 |
| Volume macroporeux (mL/g) | 0,02 | 0,03 | 0,03 |
| Volume macroporeux (% du volume poreux total) | 2 | 5 | 5 |
| Volume microporeux (mL/g) | 0 | 0 | 0 |

Le catalyseur A est ensuite préparé par imprégnation à sec de la solution S de précurseurs de Ni, décrite dans l'exemple 1, sur l'alumine A1 selon les trois étapes, nommées ci-dessous h) à i) :
h) Une imprégnation à sec de l'alumine A1 en ajoutant goutte-à-goutte un volume de 11,5 mL de solution S sur une masse de 10,5 g d'alumine A1, pendant une durée de 10 minutes.
i) Un séchage du précurseur catalytique obtenu à l'issu de l'étape h) en étuve à 120°C pendant une nuit.
j) Un traitement thermique par calcination du catalyseur séché sous un flux d'air de 1 L/h/g de catalyseur, à 450 °C pendant 2 heures (rampe de montée en température de 5 °C/min). On obtient alors le catalyseur calciné A.

Les caractéristiques du catalyseur calciné A ainsi obtenu sont reportées dans le Tableau 2 ci-après. Le catalyseur A calciné ne contient pas de pores de diamètre comprise entre 2 et 7 nm.

**Tableau 2 : Propriétés des catalyseurs A (selon l'invention), et B et C (comparatifs)**

| CATALYSEURS | A | B | C |
|---|---|---|---|
| | Selon l'invention | Comparatif | Comparatif |
| Ni (%pds) | 20,3 | 21,5 | 21,0 |
| Surface B.E.T. (m²/g) | 117 | 188 | 206 |
| Volume poreux total (mL/g) | 0,64 | 0,44 | 0,43 |
| Volume mésoporeux (mL/g) | 0,62 | 0,41 | 0,40 |
| Diamètre médian mésoporeux (nm) | 19 | 9 | 16 |
| Volume macroporeux (mL/g) | 0,02 | 0,03 | 0,03 |
| Volume macroporeux (% du volume poreux total) | 3 | 7 | 7 |
| Volume microporeux (mL/g) | 0 | 0 | 0 |
| Taille des cristallites de NiO (nm) | 15,2 | 14,1 | 11,0 |

### Exemple 3 : Préparation du catalyseur B ayant une répartition poreuse différente (comparatif)

Le catalyseur B est préparé par imprégnation à sec de la solution S de précurseurs de Ni en visant une teneur de 20 % poids en nickel par rapport à la masse totale du catalyseur sur une alumine B1 ayant une répartition poreuse différente de celle de l'alumine A1 décrite dans l'exemple 2 ci-dessus. Les caractéristiques de cette alumine B1 sont reportées dans le Tableau 1. En particulier, cette alumine B1 présente un diamètre médian mésoporeux très inférieur à celui de l'alumine A1 ainsi qu'un volume poreux total et un volume mésoporeux inférieurs à ceux de l'alumine A1.

Le catalyseur B est ensuite préparé par imprégnation à sec de la solution S de précurseurs de Ni, décrite dans l'exemple 1, sur l'alumine B1. Sur cette alumine de faible volume poreux, deux imprégnations successives ont été nécessaires pour atteindre une teneur en Ni de 20 % poids environ ; l'enchainement des trois étapes h) à i) de l'exemple 2 décrit ci-dessus, a alors été répété deux fois (selon la séquence h, i, j, h, i, j). La première étape d'imprégnation permet d'obtenir une teneur de 14,9 % poids en nickel, la deuxième étape d'imprégnation permet d'atteindre une teneur de 21,5 % poids en nickel par rapport à la masse totale du catalyseur. Pour chaque étape, les conditions opératoires sont strictement identiques à celles décrites dans l'exemple 2 ci-dessus. On obtient alors le catalyseur calciné B.

Les caractéristiques du catalyseur calciné B ainsi obtenu sont reportées dans le Tableau 2. Il présente un diamètre médian mésoporeux très inférieur à celui du catalyseur A, ainsi qu'un volume poreux total, un volume mésoporeux et des cristallites de NiO plus petits que ceux du catalyseur A.

### Exemple 4 : Préparation du catalyseur C ayant une répartition poreuse différente (comparatif)

Le catalyseur C est préparé par imprégnation à sec de la solution S de précurseurs de Ni en visant une teneur de 20 % poids en nickel par rapport à la masse totale du catalyseur sur une alumine C1 ayant une répartition poreuse encore différente de celle de l'alumine A1 décrite dans l'exemple 2 ci-dessus. Les caractéristiques de cette alumine C1 sont reportées dans le Tableau 1. En particulier, cette alumine C1 présente pas un volume poreux total et un volume mésoporeux inférieurs à ceux de l'alumine A1 mais un diamètre médian mésoporeux proche de celui de l'alumine A1. Le catalyseur C est ensuite préparé par imprégnation à sec de la solution S de précurseurs de Ni, décrite dans l'exemple 1, sur l'alumine C1. Sur cette alumine de faible volume poreux, deux imprégnations successives ont été nécessaires pour atteindre une teneur en Ni de 20 % poids environ ; l'enchainement des trois étapes h) à i) de l'exemple 2 décrit ci-dessus, a alors été répété deux fois (selon la séquence h, i, j, h, i, j). La première étape d'imprégnation permet d'obtenir une teneur de 13,3 % poids en nickel, la deuxième étape d'imprégnation permet d'atteindre une teneur de 21,0 % poids en nickel par rapport à la masse totale du catalyseur. Pour chaque étape, les conditions opératoires sont strictement identiques à celles décrites dans l'exemple 2 ci-dessus. On obtient alors le catalyseur calciné C.

Les caractéristiques du catalyseur calciné C ainsi obtenu sont reportées dans le Tableau 2. Il présente un volume poreux total et un volume mésoporeux et des cristallites de NiO plus petits que ceux du catalyseur A.

### Exemple 5 : Évaluation des propriétés catalytiques des catalyseurs A, B et C en hydrogénation sélective d'un mélange contenant du styrène et de l'isoprène

Les catalyseurs A, B et C décrits dans les exemples ci-dessus sont testés vis-à-vis de la réaction d'hydrogénation sélective d'un mélange contenant du styrène et de l'isoprène.

La composition de la charge à hydrogéner sélectivement est la suivante : 8 %pds styrène (fournisseur Sigma Aldrich®, pureté 99%), 8 %pds isoprène (fournisseur Sigma Aldrich®, pureté 99%), 84 %pds n-heptane (solvant) (fournisseur VWR®, pureté > 99% chromanorm HPLC). Cette charge contient également des composés soufrés en très faible teneur: 10 ppm pds de soufre introduits sous forme de pentanethiol (fournisseur Fluka®, pureté > 97%) et 100 ppm pds de soufre introduits sous forme de thiophène (fournisseur Merck®, pureté 99%). Cette composition correspond à la composition initiale du mélange réactionnel. Ce mélange de molécules modèles est représentatif d'une essence de pyrolyse.

La réaction d'hydrogénation sélective est opérée dans un autoclave de 500 mL en acier inoxydable, muni d'une agitation mécanique à entraînement magnétique et pouvant fonctionner sous une pression maximale de 100 bar (10 MPa) et des températures comprises entre 5°C et 200°C.

Préalablement à son introduction dans l'autoclave, une quantité de 3 mL de catalyseur est réduite *ex situ* sous un flux d'hydrogène de 1 L/h/g de catalyseur, à 400 °C pendant 16 heures (rampe de montée en température de 1 °C/min), puis elle est transvasée dans l'autoclave, à l'abri de l'air. Après ajout de 214 mL de n-heptane (fournisseur VWR®, pureté > 99% chromanorm HPLC), l'autoclave est fermé, purgé, puis pressurisé sous 35 bar (3,5 MPa) d'hydrogène, et porté à la température du test égale à 30°C. Au temps t=0, environ 30 g d'un mélange contenant du styrène, de l'isoprène, du n-heptane, du pentanethiol et du thiophène sont introduits dans l'autoclave. Le mélange réactionnel a alors la composition décrite ci-dessus et l'agitation est mise en route à 1600 tr/min. La pression est maintenue constante à 35 bar (3,5 MPa) dans l'autoclave à l'aide d'une bouteille réservoir située en amont du réacteur.

L'avancement de la réaction est suivi par prélèvement d'échantillons du milieu réactionnel à intervalles de temps réguliers : le styrène est hydrogéné en éthylbenzène, sans hydrogénation du cycle aromatique, et l'isoprène est hydrogéné en méthyl-butènes. Si la réaction est prolongée plus longtemps que nécessaire, les méthyl-butènes sont à leur tour hydrogénés en isopentane. La consommation d'hydrogène est également suivie au cours du temps par la diminution de pression dans une bouteille réservoir située en amont du réacteur. L'activité catalytique est exprimée en moles de H₂ consommées par minute et par gramme de Ni.

Les activités catalytiques mesurées pour les catalyseurs A, B et C sont reportées dans le Tableau 3 ci-dessous. Elles sont rapportées à l'activité catalytique mesurée pour le catalyseur A (A_{HYD1}).

**Tableau 3 : Comparaison des performances en hydrogénation sélective d'un mélange contenant du styrène et de l'isoprène (A_{HYD1}) et en hydrogénation du toluène (A_{HYD2}).**

| Catalyseur | Conforme ? | Taille des cristallites de NiO (nm) | A_{HYD1} (%) | A_{HYD2} (%) |
|---|---|---|---|---|
| A | Oui | 15,2 | 100 | 100 |
| B | Non | 14,1 | 48 | 41 |
| C | Non | 11,0 | 63 | 54 |

Ceci montre bien les performances améliorées du catalyseur A préparé selon l'invention et en particulier l'impact de ses propriétés texturales spécifiques. En effet, les catalyseurs B et C, bien qu'ayant des cristallites de NiO de taille inférieure à celles du catalyseur A, ont des performances catalytiques moins bonnes. La présence de mésopores de taille contrôlée est donc nécessaire pour obtenir les performances améliorées du catalyseur A.

### Exemple 6 : Évaluation des propriétés catalytiques des catalyseurs A, B et C en hydrogénation du toluène

Les catalyseurs A, B et C décrits dans les exemples ci-dessus sont également testés vis-à-vis de la réaction d'hydrogénation du toluène.

La réaction d'hydrogénation sélective est opérée dans le même autoclave que celui décrit à l'exemple 5.

Préalablement à son introduction dans l'autoclave, une quantité de 2 mL de catalyseur est réduite *ex situ* sous un flux d'hydrogène de 1 L/h/g de catalyseur, à 400 °C pendant 16 heures (rampe de montée en température de 1 °C/min), puis elle est transvasée dans l'autoclave, à l'abri de l'air. Après ajout de 216 mL de n-heptane (fournisseur VWR®, pureté > 99% chromanorm HPLC), l'autoclave est fermé, purgé, puis pressurisé sous 35 bar (3,5 MPa) d'hydrogène, et porté à la température du test égale à 80°C. Au temps t=0, environ 26 g de toluène (fournisseur SDS®, pureté > 99.8%) sont introduits dans l'autoclave (la composition initiale du mélange réactionnel est alors toluène 6 %pds / n-heptane 94 %pds) et l'agitation est mise en route à 1600 tr/min. La pression est maintenue constante à 35 bar (3,5 MPa) dans l'autoclave à l'aide d'une bouteille réservoir située en amont du réacteur.

L'avancement de la réaction est suivi par prélèvement d'échantillons du milieu réactionnel à intervalles de temps réguliers : le toluène est totalement hydrogéné en méthylcyclohexane. La consommation d'hydrogène est également suivie au cours du temps par la diminution de pression dans une bouteille réservoir située en amont du réacteur. L'activité catalytique est exprimée en moles de H₂ consommées par minute et par gramme de Ni.

Les activités catalytiques mesurées pour les catalyseurs A, B et C sont reportées dans le Tableau 3. Elles sont rapportées à l'activité catalytique mesurée pour le catalyseur A (A_{HYD2}). On retrouve les performances améliorées du catalyseur A préparé selon l'invention.

## Revendications

1. Catalyseur supporté comprenant un support oxyde majoritairement aluminique calciné et une phase active comprenant du nickel, la teneur en nickel, mesurée par fluorescence X, étant comprise entre 5 et 65 % poids dudit élément par rapport à la masse totale du catalyseur, ladite phase active ne comprenant pas de métal du groupe VIB, ledit support a une teneur en alumine calcinée supérieure ou égale à 90% poids par rapport au poids total de ladite matrice, éventuellement complétée par de la silice et/ou du phosphore à une teneur totale d'au plus 10% poids en équivalent SiO₂ et/ou P₂O₅, par rapport au poids total de ladite matrice, les particules de nickel, déterminé par diffraction des rayons X, ayant un diamètre inférieur ou égal à 20 nm, ledit catalyseur ayant un diamètre médian mésoporeux, tel que mesuré par intrusion au porosimètre à mercure, compris entre 18 et 25 nm, un volume mésoporeux mesuré par porosimétrie au mercure supérieur ou égal à 0,45 mL/g, un volume poreux total mesuré par porosimétrie au mercure supérieur ou égal à 0,45 mL/g, un volume macroporeux inférieur à 5 % du volume poreux total, ledit volume mésoporeux, le volume poreux total et le volume macroporeux étant mesurés par porosité au mercure selon la norme ASTM D4284-83, ledit catalyseur étant sous forme de grains ayant un diamètre moyen compris entre 0,5 et 10 mm.

2. Catalyseur selon la revendication 1, dans lequel le volume mésoporeux du catalyseur est compris entre 0,55 mL/g et 0,95 mL/g.

3. Catalyseur selon les revendications 1 à 2, dans lequel le volume macroporeux du catalyseur est inférieur à 3,5 % du volume poreux total.

4. Catalyseur selon les revendications 1 à 3, dans lequel la teneur en nickel est comprise entre 10 et 34 % poids dudit élément par rapport à la masse totale du catalyseur.

5. Catalyseur selon les revendications 1 à 4, lequel ne contenant pas de pores de diamètre compris entre 2 et 7 nm.

6. Catalyseur selon les revendications 1 à 5, lequel ne contient pas de micropores.

7. Procédé de préparation d'un catalyseur selon les revendications 1 à 6, comportant les étapes suivantes :
a) une première étape de précipitation, en milieu réactionnel aqueux, d'au moins un précurseur basique choisi parmi l'aluminate de sodium, l'aluminate de potassium, l'ammoniaque, l'hydroxyde de sodium et l'hydroxyde de potassium et d'au moins un précurseur acide choisi parmi le sulfate d'aluminium, le chlorure d'aluminium, le nitrate d'aluminium, l'acide sulfurique, l'acide chlorhydrique et l'acide nitrique, dans lequel au moins un des précurseurs basique ou acide comprend de l'aluminium, le débit relatif des précurseurs acide et basique est choisi de manière à obtenir un pH du milieu réactionnel compris entre 8,5 et 10,5 et le débit du ou des précurseurs acide et basique contenant de l'aluminium est réglé de manière à obtenir un taux d'avancement de la première étape compris entre 5 et 13%, le taux d'avancement étant défini comme étant la proportion d'alumine formée en équivalent Al₂O₃ lors de ladite première étape de précipitation par rapport à la quantité totale d'alumine formée en équivalent Al₂O₃ à l'issue de l'étape c) du procédé de préparation, ladite étape opérant à une température comprise entre 20 et 90°C et pendant une durée comprise entre 2 et 30 minutes,
b) une étape de chauffage de la suspension obtenue à l'étape a) à une température comprise entre 40 et 90°C pendant une durée comprise entre 7 et 45 minutes pour obtenir un gel d'alumine,
c) une deuxième étape de précipitation de la suspension obtenue à l'issue de l'étape de chauffage b) par ajout dans la suspension d'au moins un précurseur basique choisi parmi l'aluminate de sodium, l'aluminate de potassium, l'ammoniaque, l'hydroxyde de sodium et l'hydroxyde de potassium et d'au moins un précurseur acide choisi parmi le sulfate d'aluminium, le chlorure d'aluminium, le nitrate d'aluminium, l'acide sulfurique, l'acide chlorhydrique et l'acide nitrique, dans lequel au moins un des précurseurs basique ou acide comprend de l'aluminium, le débit relatif des précurseurs acide et basique est choisi de manière à obtenir un pH du milieu réactionnel compris entre 8,5 et 10,5 et le débit du ou des précurseurs acide et basique contenant de l'aluminium est réglé de manière à obtenir un taux d'avancement de la deuxième étape compris entre 87 et 95%, le taux d'avancement étant défini comme étant la proportion d'alumine formée en équivalent Al₂O₃ lors de ladite deuxième étape de précipitation par rapport à la quantité totale d'alumine formée en équivalent Al₂O₃ à l'issue de l'étape c) du procédé de préparation, ladite étape opérant à une température comprise entre 40 et 90°C et pendant une durée comprise entre 2 et 50 minutes,
d) une étape de filtration de la suspension obtenue à l'issue de l'étape c) de deuxième précipitation pour obtenir un gel d'alumine,
e) une étape de séchage dudit gel d'alumine obtenu à l'étape d) pour obtenir une poudre,
f) une étape de mise en forme de la poudre obtenue à l'issue de l'étape e) pour obtenir un matériau cru,
g) une étape de traitement thermique du matériau cru obtenu à l'issue de l'étape f) à une température comprise entre 500 et 1000°C, en présence ou non d'un flux d'air contenant jusqu'à 60% en volume d'eau, pour obtenir un support oxyde aluminique calciné,
h) une étape d'imprégnation dudit support avec une solution comprenant le ou les sel(s) du ou des précurseur(s) de la phase active à base de nickel,
i) une étape de séchage dudit support imprégné à une température comprise entre 15 et inférieure à 250°C, de manière à obtenir un catalyseur séché,
j) éventuellement un traitement thermique dudit catalyseur séché à une température comprise entre 250 et 1000°C en présence ou non d'eau.

8. Procédé selon la revendication 7, dans lequel on effectue au moins une étape de traitement réducteur k) en présence d'un gaz réducteur après les étapes i) ou j) de manière à obtenir un catalyseur comprenant du nickel au moins partiellement sous forme métallique.

9. Procédé selon la revendication 8, dans lequel on effectue une étape de passivation l) par un composé soufré ou oxygéné ou par le CO₂ avant ou après l'étape de traitement réducteur k).

10. Procédé selon les revendications 7 à 9, dans lequel le taux d'avancement de la première étape de précipitation a) est compris entre 6 et 12%.

11. Procédé selon les revendications 7 à 10, dans lequel le précurseur acide des étapes a) et c) est choisi parmi le sulfate d'aluminium, le chlorure d'aluminium et le nitrate d'aluminium et dans lequel le précurseur basique des étapes a) et c) est choisi parmi l'aluminate de sodium et l'aluminate de potassium.

12. Procédé d'hydrogénation dans lequel le catalyseur selon les revendications 1 à 6 est mis en contact en présence d'hydrogène avec une charge d'hydrocarbures contenant des composés polyinsaturés et/ou aromatiques de manière à obtenir un effluent au moins partiellement hydrogéné et
dans lequel on effectue une hydrogénation sélective à une température comprise entre 0 et 500°C, à une pression comprise entre 0,1 et 20 MPa, à un ratio molaire hydrogène/(composés polyinsaturés à hydrogéner) entre 0,1 et 10 et à une vitesse volumique horaire comprise entre 0,1 et 200 h⁻¹ pour une charge liquide, comprise entre 100 et 15000 h⁻¹ pour une charge gazeuse, d'une charge d'hydrocarbures contenant des composés polyinsaturés contenant au moins 2 atomes de carbone par molécule et ayant un point d'ébullition final inférieur ou égal à 250°C ou on effectue une hydrogénation des aromatiques à une température comprise entre 30 et 350°C, à une pression comprise entre 0,1 et 20 MPa, à un ratio molaire hydrogène/(composés aromatiques à hydrogéner) entre 0,1 et 10 et à une vitesse volumique horaire comprise entre 0,05 et 50 h⁻¹, d'une charge d'hydrocarbures contenant des composés aromatiques et ayant un point d'ébullition final inférieur ou égal à 650°C.

## Patentansprüche

1. Geträgerter Katalysator, umfassend einen kalzinierten Träger, der überwiegend aus Aluminiumoxid besteht, und eine aktive Phase, die Nickel umfasst, wobei der Nickelgehalt, gemessen mittels Fluoreszenz X, zwischen 5 und 65 Gew.-% des Elements, bezogen auf das Gesamtgewicht des Katalysators, liegt, wobei die aktive Phase kein Metall der Gruppe VIB umfasst, wobei der Träger einen Gehalt an kalziniertem Aluminiumoxid größer oder gleich 90 Gew.-% aufweist, bezogen auf das Gesamtgewicht dieser Matrix, gegebenenfalls ergänzt mit Siliziumdioxid und/oder Phosphor mit einem Gesamtgehalt von höchstens 10 Gew.-% als SiO₂- und/oder P₂O₅-Äquivalent, bezogen auf das Gesamtgewicht der Matrix, wobei die Nickelpartikel, bestimmt mittels Röntgenbeugung, einen Durchmesser kleiner oder gleich 20 nm aufweisen, wobei der Katalysator einen mittleren Mesoporendurchmesser, gemessen mittels Quecksilber-Intrusionsporosimetrie, im Bereich zwischen 18 und 25 nm aufweist, ein Mesoporenvolumen, gemessen mittels Quecksilberporosimetrie, größer oder gleich 0,45 ml/g, ein Gesamtporenvolumen, gemessen mittels Quecksilberporosimetrie, größer oder gleich 0,45 ml/g, ein Makroporenvolumen von weniger als 5 % des Gesamtporenvolumens, wobei das Mesoporenvolumen, des Gesamtporenvolumen und das Makroporenvolumen mittels Quecksilberporosimetrie gemäß der Norm ASTM D4284-83 gemessen werden, wobei der Katalysator in Form von Körnern vorliegt, die einen mittleren Durchmesser im Bereich zwischen 0,5 und 10 mm aufweisen.

2. Katalysator nach Anspruch 1, wobei das Mesoporenvolumen des Katalysators im Bereich zwischen 0,55 ml/g und 0,95 ml/g liegt.

3. Katalysator nach den Ansprüchen 1 bis 2, wobei das Makroporenvolumen des Katalysators weniger als 3,5 % des Gesamtporenvolumens ausmacht.

4. Katalysator nach den Ansprüchen 1 bis 3, wobei der Gehalt an Nickel im Bereich zwischen 10 und 34 Gew.-% des Elements liegt, bezogen auf das Gesamtgewicht des Katalysators.

5. Katalysator nach den Ansprüchen 1 bis 4, der keine Poren mit einem Durchmesser im Bereich zwischen 2 und 7 nm enthält.

6. Katalysator nach den Ansprüchen 1 bis 5, der keine Mikroporen enthält.

7. Verfahren zur Herstellung eines Katalysators nach den Ansprüchen 1 bis 6, umfassend die folgenden Schritte:
a) einen ersten Schritt zum Ausfällen, in wässrigem Reaktionsmedium, mindestens einer basischen Vorstufe, ausgewählt aus Natriumaluminat, Kaliumaluminat, Ammoniak, Natriumhydroxid und Kaliumhydroxid, und mindestens einer sauren Vorstufe, ausgewählt aus Aluminiumsulfat, Aluminiumchlorid, Aluminiumnitrat, Schwefelsäure, Salzsäure und Salpetersäure, wobei mindestens eine der basischen oder sauren Vorstufen Aluminium umfasst, wobei der relative Durchsatz der sauren und basischen Vorstufen so ausgewählt ist, dass ein pH-Wert des Reaktionsmediums im Bereich zwischen 8,5 und 10,5 erhalten wird, und der Durchsatz der sauren und basischen Vorstufe(n), die Aluminium enthalten, so reguliert ist, dass ein Fortschrittsgrad des ersten Schritts im Bereich zwischen 5 und 13 % erhalten wird, wobei der Fortschrittsgrad als der Anteil an Aluminiumoxid, gebildet als Al₂O₃-Äquivalent während des ersten Schritts zum Ausfällen, bezogen auf die Gesamtmenge an Aluminiumoxid, gebildet als Al₂O₃-Äquivalent am Ende von Schritt c) des Herstellungsverfahrens, definiert ist, wobei der Schritt bei einer Temperatur im Bereich zwischen 20 und 90 °C und für eine Dauer im Bereich zwischen 2 und 30 Minuten durchgeführt wird,
b) einen Schritt zum Erwärmen der Suspension, die in Schritt a) erhalten wurde, auf eine Temperatur im Bereich zwischen 40 und 90 °C für eine Dauer im Bereich zwischen 7 und 45 Minuten, um ein Aluminiumoxidgel zu erhalten,
c) einen zweiten Schritt zum Ausfällen der Suspension, die am Ende des Schritts zum Erwärmen b) erhalten wird, durch Zugeben zu der Suspension mindestens einer basischen Vorstufe, ausgewählt aus Natriumaluminat, Kaliumaluminat, Ammoniak, Natriumhydroxid und Kaliumhydroxid, und mindestens einer sauren Vorstufe, ausgewählt aus Aluminiumsulfat, Aluminiumchlorid, Aluminiumnitrat, Schwefelsäure, Salzsäure und Salpetersäure, wobei mindestens eine der basischen oder sauren Vorstufen Aluminium umfasst, wobei der relative Durchsatz der sauren und basischen Vorstufen so ausgewählt ist, dass ein pH-Wert des Reaktionsmediums im Bereich zwischen 8,5 und 10,5 erhalten wird, und der Durchsatz der sauren und basischen Vorstufe(n), die Aluminium enthalten, so reguliert ist, dass ein Fortschrittsgrad des zweiten Schritts im Bereich zwischen 87 und 95 % erhalten wird, wobei der Fortschrittsgrad als der Anteil an Aluminiumoxid, gebildet als Al₂O₃-Äquivalent während des zweiten Fällungsschritts, bezogen auf die Gesamtmenge an Aluminiumoxid, gebildet als Al₂O₃-Äquivalent am Ende von Schritt c) des Herstellungsverfahrens, definiert ist, wobei der Schritt bei einer Temperatur im Bereich zwischen 40 und 90 °C und für eine Dauer im Bereich zwischen 2 und 50 Minuten durchgeführt wird,
d) einen Schritt zum Filtrieren der Suspension, die am Ende von Schritt c) zur zweiten Ausfällung erhalten wurde, um ein Aluminiumoxidgel zu erhalten,
e) einen Schritt zum Trocknen des Aluminiumoxidgels, das in Schritt d) erhalten wird, um ein Pulver zu erhalten,
f) einen Schritt zum Formen des Pulvers, das am Ende von Schritt e) erhalten wird, um ein Rohmaterial zu erhalten;
g) einen Schritt zur Wärmebehandlung des Rohmaterials, das am Ende von Schritt f) erhalten wird, bei einer Temperatur im Bereich zwischen 500 und 1.000 °C, in Gegenwart eines Luftstroms oder nicht, der bis zu 60 Vol.-% Wasser enthält, um einen kalzinierten Aluminiumoxidträger zu erhalten;
h) einen Schritt zum Imprägnieren des Trägers mit einer Lösung, umfassend das oder die Salz(e) der Vorstufe(n) der aktiven Phase auf Nickelbasis,
i) einen Schritt zum Trocknen des imprägnierten Träges bei einer Temperatur im Bereich zwischen 15 und weniger als 250 °C, um einen getrockneten Katalysator zu erhalten,
j) gegebenenfalls eine Wärmebehandlung des getrockneten Katalysators bei einer Temperatur im Bereich zwischen 250 und 1.000 °C mit oder ohne Wasser.

8. Verfahren nach Anspruch 7, wobei mindestens ein Schritt zur Reduktionsbehandlung k) in Gegenwart eines Reduktionsgases nach den Schritten i) oder j) durchgeführt wird, um einen Katalysator zu erhalten, der Nickel mindestens teilweise in metallischer Form umfasst.

9. Verfahren nach Anspruch 8, wobei ein Passivierungsschritt l) mit einer Schwefel- oder Sauerstoffverbindung oder mit CO₂ vor oder nach dem Reduktionsbehandlungsschritt k) durchgeführt wird.

10. Verfahren nach den Ansprüchen 7 bis 9, wobei der Fortschrittsgrad des ersten Schritts zum Ausfällen a) im Bereich zwischen 6 und 12 % liegt.

11. Verfahren nach den Ansprüchen 7 bis 10, wobei die saure Vorstufe der Schritte a) und c) ausgewählt ist aus Aluminiumsulfat, Aluminiumchlorid und Aluminiumnitrat und wobei die basische Vorstufe der Schritte a) und c) ausgewählt ist aus Natriumaluminat und Kaliumaluminat.

12. Hydrierverfahren, wobei der Katalysator nach den Ansprüchen 1 bis 6 in Gegenwart von Wasserstoff mit einem Kohlenwasserstoffeinsatzmaterial in Kontakt gebracht wird, das mehrfach ungesättigte und/oder aromatische Verbindungen enthält, um einen Abstrom zu erhalten, der mindestens teilweise hydriert ist, und wobei eine selektive Hydrierung bei einer Temperatur im Bereich zwischen 0 und 500 °C, bei einem Druck im Bereich zwischen 0,1 und 20 MPa, einem Molverhältnis von Wasserstoff/(zu hydrierende mehrfach ungesättigte Verbindungen) zwischen 0,1 und 10 und bei einer Raumgeschwindigkeit pro Stunde im Bereich zwischen 0,1 und 200 h⁻¹ für ein flüssiges Einsatzmaterial, im Bereich zwischen 100 und 15.000 h⁻¹ für ein gasförmiges Einsatzmaterial, eines Kohlenwasserstoffeinsatzmaterial, das mehrfach ungesättigte Verbindungen enthält, die mindestens 2 Kohlenstoffatome pro Molekül enthalten und einen Endsiedepunkt kleiner oder gleich 250 °C aufweist, durchgeführt wird, oder eine Hydrierung der Aromaten bei einer Temperatur im Bereich zwischen 30 und 350 °C, bei einem Druck im Bereich zwischen 0,1 und 20 MPa, bei einem Molverhältnis Wasserstoff/(zu hydrierende aromatische Verbindungen) zwischen 0,1 und 10 und bei einer Raumgeschwindigkeit pro Stunde im Bereich zwischen 0,05 und 50 h⁻¹, eines Kohlenwasserstoffeinsatzmaterials, das aromatische Verbindungen enthält und das einen Endsiedpunkt kleiner oder gleich 650 °C aufweist, durchgeführt wird.

## Claims

1. Supported catalyst comprising a calcined, predominantly aluminium, oxide support and an active phase comprising nickel, the nickel content, measured by X-ray fluorescence, being comprised between 5 and 65% by weight of said element with respect to the total mass of the catalyst, said active phase comprising no group VIB metal, comprising a content of alumina greater than or equal to 90% and a content of silica as SiO₂ equivalent of at most 10% with respect to the final oxide, optionally completed by silica and/or phosphorus with a total content of at most 10% by weight of an equivalent of SiO₂ and/or P₂O₅ with respect to the final oxide, the nickel particles, determined by X-ray diffraction, having a diameter less than or equal to 20 nm, said catalyst having a mesopore median diameter, measured by mercury intrusion porosimetry, between 18 and 25 nm, a mesopore volume measured by mercury porosimetry greater than or equal to 0.45 mUg, a total pore volume measured by mercury porosimetry greater than or equal to 0.45 mL/g, a macropore volume less than 5% of the total pore volume, said mesopore volume, said total pore volume and said macropore volume being measured by mercury intrusion porosimetry according to ASTM standard D4284-83, said catalyst being in the form of grains having an average diameter comprised between 0.5 and 10 mm.

2. Catalysts according to claim 1, in which the mesopore volume of the catalyst is comprised between 0.55 ml/g and 0.95 ml/g.

3. Catalyst according to claims 1 or 2, in which the macropore volume of the catalyst is less than 3.5% of the total pore volume.

4. Catalyst according to claims 1 to 3, in which the nickel content is comprised between 10 and 34% by weight of said element with respect to the total mass of the catalyst.

5. Catalyst according to claims 1 to 4, containing no pores with a diameter comprised between 2 and 7 nm.

6. Catalyst according to claims 1 to 5, containing no micropores.

7. Process for the preparation of a catalyst according to one of claims 1 to 6 comprising the following steps:
a) a first step of precipitation, in an aqueous reaction medium, of at least one basic precursor selected from sodium aluminate, potassium aluminate, ammonium hydroxide, sodium hydroxide and potassium hydroxide and of at least one acidic precursor selected from aluminium sulphate, aluminium chloride, aluminium nitrate, sulphuric acid, hydrochloric acid and nitric acid, in which at least one of the basic or acidic precursors comprises aluminium, the relative flow rate of the acidic and basic precursors is selected so as to obtain a pH of the reaction medium comprised between 8.5 and 10.5 and the flow rate of the acidic and basic precursor(s) containing aluminium is adjusted so as to obtain a degree of progression of the first step comprised between 5 and 13%, the degree of progression being defined as the proportion of alumina formed in Al₂O₃ equivalent during said first step of precipitation with respect to the total quantity of alumina formed in Al₂O₃ equivalent at the end of step c) of the preparation process, said step taking place at a temperature comprised between 20 and 90°C and for a duration comprised between 2 and 30 minutes;
b) a step of heating the suspension obtained in step a) at a temperature comprised between 40 and 90°C for a duration comprised between 7 and 45 minutes in order to obtain an alumina gel,
c) a second step of precipitation of the suspension obtained at the end of heating step b) by adding, to the suspension, at least one basic precursor selected from sodium aluminate, potassium aluminate, ammonium hydroxide, sodium hydroxide and potassium hydroxide and at least one acidic precursor selected from aluminium sulphate, aluminium chloride, aluminium nitrate, sulphuric acid, hydrochloric acid and nitric acid, in which at least one of the basic or acidic precursors comprises aluminium, the relative flow rate of the acidic and basic precursors is selected so as to obtain a pH of the reaction medium comprised between 8.5 and 10.5 and the flow rate of the acidic and basic precursor(s) containing aluminium is adjusted so as to obtain a degree of progression of the second step comprised between 87 and 95%, the degree of progression being defined as the proportion of alumina formed in Al₂O₃ equivalent during said second precipitation step with respect to the total quantity of alumina formed in Al₂O₃ equivalent at the end of step c) of the preparation process, said step taking place at a temperature comprised between 40 and 90°C and for a duration comprised between 2 and 50 minutes;
d) a step of filtration of the suspension obtained at the end of second precipitation step c) in order to obtain an alumina gel,
e) a step of drying said alumina gel obtained in step d) in order to obtain a powder,
f) a step of forming the powder obtained at the end of step e) in order to obtain a crude material,
g) a step of heat treatment of the crude material obtained at the end of step f) at a temperature comprised between 500 and 1000°C, in the presence or absence of an air flow containing up to 60% by volume of water, in order to obtain a calcined aluminium oxide support;
h) a step of impregnation of said support with a solution comprising the salt(s) of the precursor(s) of the nickel-based active phase;
i) a step of drying said impregnated support at a temperature comprised between 15 and 250°C so as to obtain a dried catalyst,
j) optionally a heat treatment of said dried catalyst at a temperature comprised between 250 and 1000°C, in the presence or absence of water.

8. Process according to claim 7, in which at least one reducing treatment step k) is carried out in the presence of a reducing gas after steps i) or j) so as to obtain a catalyst comprising nickel at least partially in metallic form.

9. Process according to claim 8, in which a passivation step I) is carried out with a sulphur- or oxygen-containing compound, or with CO₂ before or after reducing treatment step k).

10. Process according to claims 7 to 9, in which the degree of progression of the first precipitation step a) is comprised between 6 and 12%.

11. Process according to claims 7 to 10, in which the acidic precursor of steps a) and c) is selected from aluminium sulphate, aluminium chloride and aluminium nitrate, and in which the basic precursor of steps a) and c) is selected from sodium aluminate and potassium aluminate.

12. Hydrogenation process in which the catalyst according to claims 1 to 6 is brought into contact, in the presence of hydrogen, with a feedstock of hydrocarbons containing polyunsaturated and/or aromatic compounds so as to obtain an at least partially hydrogenated effluent and
in which a selective hydrogenation is carried out at a temperature comprised between 0 and 500°C, at a pressure comprised between 0.1 and 20 MPa, at a hydrogen/(polyunsaturated compounds to be hydrogenated) molar ratio between 0.1 and 10 at an hourly space velocity HSV comprised between 0.1 and 200 h⁻¹ for a liquid feedstock, comprised between 100 and 15,000 h⁻¹ for a gaseous feedstock, of a hydrocarbon feedstock containing polyunsaturated compounds containing at least 2 carbon atoms per molecule and having a final boiling point less than or equal to 250°C or
in which a hydrogenation of the aromatics is carried out at a temperature comprised between 30 and 350°C, at a pressure comprised between 0.1 and 20 MPa, at a hydrogen/(aromatic compounds to be hydrogenated) molar ratio between 0.1 and 10 and at an hourly space velocity HSV comprised between 0.05 and 50 h⁻¹, of a hydrocarbon feedstock containing aromatic compounds and having a final boiling point less than or equal to 650°C.
